# EUROPEAN PATENT APPLICATION

(11) **EP 2 072 050 A1**
(43) Date of publication of application: **24.06.2009**
(21) Application number: 07025028.7
(22) Date of filing: 21.12.2007
(51) Int. Cl.: A61K 31/4545, A61P 1/08

(54) **Compounds with anti-emetic effect**

(71) Applicant: Santhera Pharmaceuticals (Schweiz) AG, 4410 Liestal (CH)
(72) Inventor: Mondadori, Cesare, 4153 Reinach (CH)
(74) Representative: Grünecker, Kinkeldey, Stockmair & Schwanhäusser Anwaltssozietät

(57) **Abstract**

The present invention relates to the use of melanocortin-4 receptor antagonists in the treatment of emesis and nausea.

## Description

### Field of the Invention

The present invention relates to the use of MC-4 receptor antagonists in the treatment of emesis and nausea.

### Background of the invention

Nausea, vomiting, and retching are basic human protective reflexes against the absorption of toxins as well as responses to certain external and internal stimuli.

Nausea is a subjectively unpleasant wavelike sensation in the back of the throat or epigastrium associated with pallor or flushing, tachycardia, and an awareness of the urge to vomit. Sweating, excess salivation, and a sensation of being cold or hot may also occur. Vomiting is characterized by contraction of the abdominal muscles, descent of the diaphragm, and opening of the gastric cardia, resulting in forceful expulsion of stomach contents from the mouth. Retching involves spasmodic contractions of the diaphragm and the muscles of the thorax and abdominal wall without expulsion of gastric contents. Emesis is used herein to refer to nausea, vomiting, and/or retching.

The vomiting reflex can be triggered by the activation of a nucleus of neurons located in the medulla oblongata (vomiting center). The various ways via which the vomiting center can be activated include
- direct signals from the cerebral cortex (anticipation, fear, memory),
- signals from sensory organs (disturbing sights, smells, pain),
- signals from the vestibular apparatus of the inner ear (motion sickness).

There is also a possibility to directly activate the vomiting center through
- activation of the chemoreceptor trigger zone (CTZ).
The CTZ is located in the highly vascular *area postrema* on the surface of the brain. Because this area lacks a true blood-brain barrier and is exposed to both blood and cerebrospinal fluid, the CTZ can react directly to substances in the blood. The CTZ can also be activated by signals from the stomach and small intestine traveling along vagal afferent nerves or by the direct action of emetogenic compounds that are carried in the blood such as, for example, chemotherapy drugs or opioids.

Specific neuronal networks in the CTZ identify substances as potentially harmful and relay impulses to the vomiting center to initiate the vomiting cascade so that the harmful substances can be expelled. The neurotransmitters critically involved in these signaling pathways include
- serotonin,
- dopamine,
- acetylcholine (muscarinic cholinergic),
- histamine
- neurokinin-1 (NK-1) neuropeptide.

Stimulation of these chemoreceptors has been found to activate the vomiting center. Many anti-emetics act by blocking one or more of these receptors. For example, dopamine antagonists presumably exert their anti-emetic effects through a blockade of dopamine receptors. Similarly the muscarinic antagonists are assumed to act through a blockade of acetylcholine receptors; the histamine antagonists through a blockade of histamine receptors etc. The main targets of anti-emetic therapy are post-operative nausea and chemotherapy-induced nausea and vomiting.

Post-operative nausea and vomiting occurs after receiving anesthesia, such as during surgery. In post-operative nausea and vomiting a wide range of stimuli contribute to the emetic response. Most anesthetic agents and opioids stimulate the vomiting center indirectly through the CTZ. Associated factors that directly stimulate the vomiting center in post-operative emesis include sensory inputs, including visual, olfactory, and pain, and the vestibular apparatus. Other anesthetics such as nitrous oxide directly stimulate the gastrointestinal tract, which may activate the vomiting center. Several classes of compounds including serotonin 5-HT3 receptor antagonists have been shown effective for the management of post-operative nausea and vomiting (Gan, CNS Drugs 2005, 19 (3), 225-238).

Chemotherapy induced nausea and vomiting exhibits several characteristic temporal patterns.
- Anticipatory emesis occurs before the beginning of a new cycle of chemotherapy in response to conditioned stimuli such as the smells, sights, and sounds of the treatment room or the presence of a specific person who administers the chemotherapy. Anticipatory emesis usually occurs several hours before administration of chemotherapy in patients who have experienced failed control of emesis.
- Acute emesis occurs within the first about 24 hours after the administration of chemotherapy.
- Delayed emesis begins at least 24 hours after initiation of chemotherapy and can last up to about 120 hours. The causative mechanism in delayed emesis is not well defined, however metabolites of an administered chemotherapeutic agent are thought to continue to affect the central nervous system and the gastrointestinal tract. For example, cisplatin causes delayed emesis up to about 48 to about 72 hours after administration in more than half of all patients who receive the drug. Breakthrough emesis occurs despite preventive therapy and requires additional anti-emetic treatment.

There are several mechanisms by which an efficient anti-emetic effect can be achieved. Chemotherapeutic agents initiate activation mainly of serotonin (5-HT) receptors, which leads to the emetic response and therefore serotonin receptor antagonists are clinically effective drugs for treating acute chemotherapy induced nausea and vomiting. Because serotonin receptor antagonists prevent emesis by blocking the emetic response early in the emetic pathway, the drugs can be given to patients before chemotherapy to prevent emesis.

Examples of clinically useful serotonin receptor antagonists include ondansetron (Zofran®), granisetron (Kytril®), dolasetron (Anzemet®), and palonosetron (Aloxi®). Because serotonin receptor antagonists are less effective in treating anticipatory, delayed, and breakthrough emesis, the drugs can also be used in combination with other anti-emetic agents to provide more comprehensive anti-emetic therapy. For example, anticipatory emesis can be treated using compounds dampening the limbic system such as lorazepam (Ativan®.), delayed emesis using corticosteroids such as dexamethasone or methylprednisolone (Solu-Medrol®.), and breakthrough emesis using dopamine receptor antagonists such as prochlorperazine (Compazine.®.), metoclopramide (Reglan®.), haloperidol (Haldol®.), or dronabinol (Marinol®.).

The need for anti-emetic agents and therapies addressing both acute and delayed emesis associated with cancer chemotherapy is highlighted by the approvals of Aloxi® and Emend®. Palonsetron (Aloxi®) is a 5-HT3 receptor antagonist with a long half-life and is the only 5-HT3 antagonist that is approved in the U.S. for the prevention of both acute and delayed emesis. Aprepitant (Emend®) is a NK-1 receptor antagonist that belongs to a new class of anti-emetic compounds and is approved for the treatment of severe and moderate chemotherapy induced emesis.

On the basis of today's experience and with regards to the anti-emetic potential, 5HT3 receptor antagonists seem to be most effective, closely followed by NK1 antagonists (Onkodin, data base, www.onkodin.de). However, the responder rates at monotherapy with NK1 and 5HT3 antagonists are not 100%, they have been found to lie somewhere between 50 and 60% at best, (Onkodin, data base, www.onkodin.de). Because of the fact that the etiology of nausea and vomiting is multifactorial, combination anti-emetic therapy is being recommended. Combinations of NK-1 or 5HT3 antagonists with steroids allow an increase of responder rate of over 80% (Onkodin, data base, www.onkodin.de). The possibilities include the use of a combination of agents acting at different receptor sites and/or a multimodal approach in which, in addition to administering anti-emetic agents that directly interfere with emetic response pathways, stimuli associated with the risk of developing emesis be minimized (see, Habib and Gan, Can. J. Anesth 2004, 51(4), 326-341). This trend towards combination therapy is also reflected in the guidelines for the prevention of chemotherapy induced emesis in patients undergoing highly emetogenic chemotherapy where the use of aprepitant (NK-1 antagonist) in combination with a 5-HT3 receptor antagonist and dexamethasone is recommend (Jordan et al. The Oncologists, Vol. 12, No. 9, 1143-1150, September 2007). However, a significant number of patients still experience emesis, indicating the need for improved anti-emetic compounds and treatments.

Thus, it is the object of the present invention to provide a new class of compounds as anti-emetica which overcome the above discussed drawbacks of the treatment presently applied.

Surprisingly, it has been found that MC-4 antagonists solve the above mentioned object.

### Summary of the invention

The present invention relates to melanocortin-4 receptor antagonists in the treatment of nausea and emesis.

MC-4 antagonists are a new class of compounds found to increase food intake in normal mice (internal observations). Positive effects on food intake strongly indicate a potential usefulness of the compounds to treat cachexia induced by cancer, inflammation and infections.

Surprisingly it was found that MC-4 antagonists also have strong anti-emetic properties.

The compounds are unique in the sense that they combine strong anti-emetic and strong anti-cachexia effects. In addition, the compounds also exhibit a strong anti-depressive effect (Shigeyuky Chaki et al, J. Pharm. Exp. Ther. (2003) 304(2), 818-826). Cachexia, emesis and depression are known side-effects in the treatment of, e.g., cancer.

This surprising finding offers the possibility to substitute the combination therapy which is presently state of the art, e.g. in the treatment of cancer, with a single drug therapy. A single drug therapy has many advantages compared to a combination therapy. It allows to avoid problems caused by PK differences between the various active substances used. By the use of only one drug for treating, for example, cachexia associated with emesis, the metabolic load may be reduced. As a further advantage, the steroid-induced muscle wasting which is a common side effect associated with the administration of steroids in the treatment of cancer can be avoided.

### Brief description of the Figures

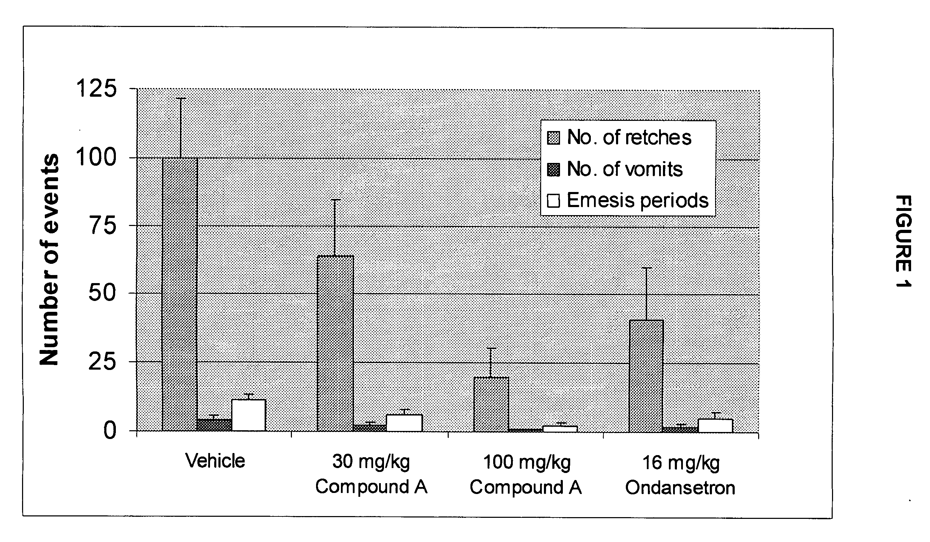

Figure 1 shows the effects of compound A and ondansetron on cisplatin induced emesis in ferrets. The histograms represent the number of retches and vomits observed over a period of 3 hours.

### Detailed description of the invention

The present invention relates to melanocortin-4 receptor antagonists for the treatment of nausea and emesis.

Nausea and emesis may occur not only in direct response of a clinical incidence but also within minutes to hours after the triggering moment has taken place. Thus, in one embodiment of the present invention, melanocortin-4 receptor antagonists are intended for the treatment of acute, delayed, post-operative, late phase and anticipatory emesis and nausea.

In a further preferred embodiment of the present invention, nausea and emesis are induced in a patient by chemotherapy.

In one preferred embodiment of the present invention, nausea and emesis are induced by dysmenorrhoe, migraine, cancer or other pain conditions.

Furthermore, in a preferred embodiment of the present invention, nausea and emesis are induced by radiation, toxins, pregnancy, alcohol withdrawal, drug withdrawal, nicotine withdrawal, vestibular disorders, motion sickness, post-operative sickness, surgery, gastrointestinal obstruction, reduced gastrointestinal mobility, visceral pain or increased or decreased intracranial pressure.

The melanocortin-4 receptor antagonists are preferably selected from the group consisting of Formulae (I) to (V): 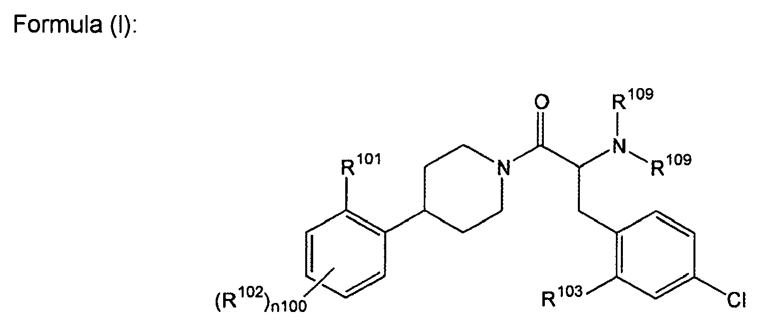 and enantiomers, diastereomers, tautomers, solvates and pharmaceutically acceptable salts thereof,
wherein
R¹⁰¹ is -(C(R¹⁰⁸)₂)ₗ₁₀₀-T¹⁰⁰,
   -O-(C(R¹⁰⁸)₂)ₘ₁₀₀-T¹⁰⁰,
T¹⁰⁰ is NR¹⁰⁵R¹⁰⁶,
   morpholine, 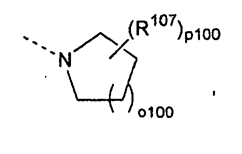 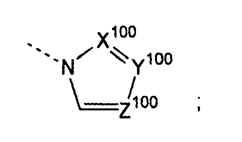
R¹⁰⁵ and R¹⁰⁶ are independently
   H
   C₁₋₆-alkyl,
   C₂₋₆-alkenyl,
   C₂₋₆-alkinyl,
   C₂₋₆-alkylene-O-C₁₋₆-alkyl
   wherein each alkyl, alkenyl and alkinyl is optionally substituted by one or
   more substituents selected from halogen atoms, CN and OH,
R¹⁰⁷ is halogen,
   CN,
   OH,
   C₁₋₆-alkyl, optionally substituted with 1 to 3 substituents selected from halogen, CN and OH,
   O-C₁₋₆-alkyl optionally substituted with 1 to 3 substituents selected from
   halogen, CN and OH,
   C₁₋₆-alkylene-O-C₁₋₆-alkyl optionally substituted with 1 to 3 substituents
   selected from halogen, CN and OH,
R¹⁰⁸ is independently
   H,
   F,
   OH,
   OMe,
   C₁₋₆-alkyl, optionally substituted with 1 to 3 substituents selected from halogen, CN, OH and OMe,
   C₃₋₆-cycloalkyl, optionally substituted with 1 to 3 substituents selected from halogen, CN, OH and OMe,
R¹⁰⁹ is independently
   H,
   C₁₋₆-alkyl, optionally substituted with 1 to 3 substituents selected from halogen, CN, OH and OMe,
   C₃₋₆-cycloalkyl, optionally substituted with 1 to 3 substituents selected from halogen, CN, OH and OMe,
X¹⁰⁰ is CH, N,
Y¹⁰⁰ is CH, N,
Z¹⁰⁰ is CH, N,
R¹⁰² is F,
   Cl,
   methyl,
R¹⁰³ is Cl,
   methyl,
l100 is 3, 4,
m100 is 2, 3, 4,
n100 is 0, 1, 2, 3, 4,
o100 is 0, 1, 2,
p100 is 0, 1, 2, 3, 4; 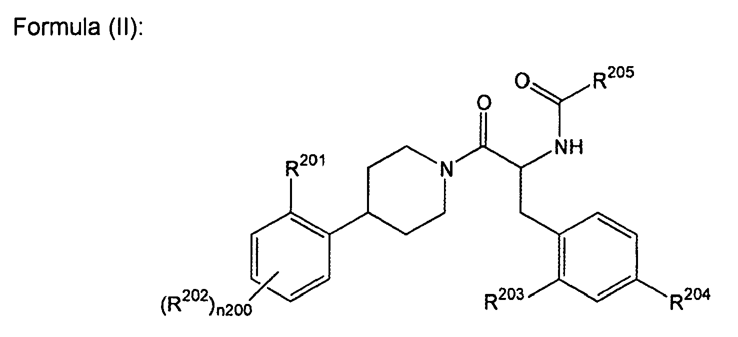 and the enantiomers, diastereomers, tautomers, solvates and pharmaceutically acceptable salts thereof,
   wherein
R²⁰¹ is -(C(R²⁰⁶)₂)ₗ₂₀₀-T²⁰⁰, or
   -O-(C(R²⁰⁶)₂)ₘ₂₀₀-T²⁰⁰;
R²⁰⁶ is independently selected from
   H,
   F,
   OH,
   OCH₃,
   C₁₋₆-alkyl, optionally substituted with 1 to 3 substituents selected from halogen, CN, OH and OCH₃, and
   C₃₋₆-cycloalkyl, optionally substituted with 1 to 3 substituents selected from halogen, CN, OH and OCH₃;
T²⁰⁰ is NR²⁰⁷R²⁰⁸,
   morpholine, 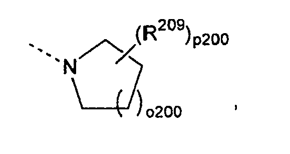 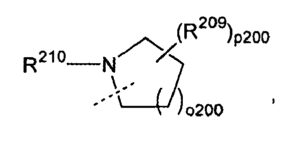 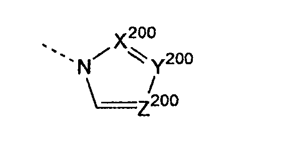 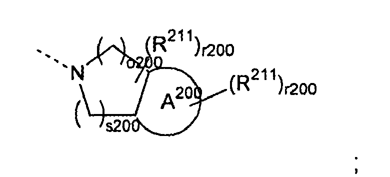
R²⁰⁷ and R²⁰⁸ are independently from each other selected from
   H,
   C₁₋₆-alkyl,
   C₂₋₆-alkenyl
   C₂₋₆-alkinyl, and
   C₂₋₆-alkylene-O-C₁₋₆-alkyl,
   wherein each alkyl, alkenyl and alkinyl is optionally substituted by one or more halogen atoms, CN or OH;
R²⁰⁹ is independently selected from
   halogen,
   CN,
   OH,
   C₁₋₆-alkyl optionally substituted with 1 to 3 substituents selected from halogen, CN and OH, and
   O-C₁₋₆-alkyl optionally substituted with 1 to 3 substituents selected from halogen, CN and OH,
   C₁₋₆-alkylene-O-C₁₋₆-alkyl optionally substituted with 1 to 3 substituents selected from halogen, CN and OH;
R²¹⁰ is H, or
   C₁-C₆-alkyl;
R²¹¹ is independently selected from
   halogen,
   CN,
   OH,
   C₁₋₆-alkyl optionally substituted with 1 to 3 substituents selected from
   halogen, CN and OH,
   O-C₁₋₆-alkyl optionally substituted with 1 to 3 substituents selected from halogen, CN and OH,
   C₁₋₆-alkylene-O-C₁₋₆-alkyl optionally substituted with 1 to 3 substituents selected from halogen, CN and OH,
   -NH₂,
   -NH(C₁₋₆-alkyl), and
   -N(C₁₋₆-alkyl)₂;
X²⁰⁰ is CH or N;
Y²⁰⁰ is CH or N;
Z²⁰⁰ is CH or N;
A²⁰⁰ is a 3-7-membered saturated, unsaturated or aromatic ring containing 0-2 nitrogen atoms;
R²⁰² is independently selected from
   F,
   Cl,
   CH₃, and
   CF_{3;}
R²⁰³ is H,
   Cl,
   F, or
   CH₃;
R²⁰⁴ is Cl or F;
R²⁰⁵ is 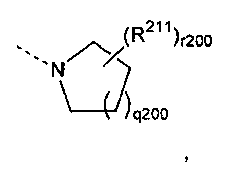 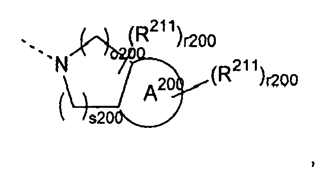 morpholine, optionally substituted by 1 to 3, same or different substituents
   R²¹⁴, or
   NR²¹²R²¹³;
R²¹² and R²¹³ are independently from each other selected from
   C₁₋₆-alkyl,
   C₂₋₆-alkenyl,
   C₂₋₆-alkinyl,
   C₂₋₆-alkylene-O-C₁₋₆-alkyl, and
   C₂₋₆-alkylene-N-(C₁₋₆-alkyl)₂;
R²¹⁴ is C₁₋₆-alkyl,
   C₁₋₆-alkylene-O-C₁₋₆-alkyl,
   C₁₋₆-alkylene-OH,
   C₁₋₆-alkylene-NH₂,
   C₁₋₆-alkylene-NH-C₁₋₆-alkyl, or
   C₁₋₆-alkylene-N(C₁₋₆-alkyl)₂;
l200 is 1, 2, 3, or 4;
m200 is 0, 1, 2, 3, or 4;
n200 is 0, 1, 2, 3, or 4;
o200 is 0, 1, or 2;
p200 is 0, 1, 2, 3, or 4;
q200 is 0, 1, 2, or 3;
r200 is 0, 1, 2, 3, or 4 and
s200 is 1, or 2; 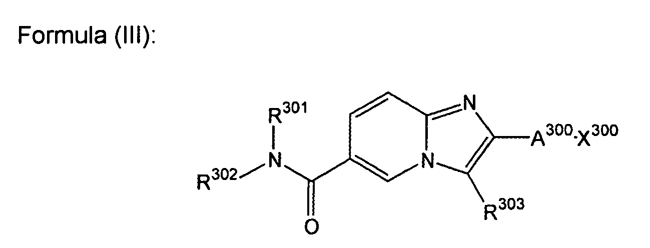 and enantiomers, diastereomers, tautomers, solvates and pharmaceutically acceptable salts thereof,
   wherein
R³⁰¹ and R³⁰² are independently from each other selected from
   H,
   C₁₋₆alkyl,
   C₁₋₆ alkylene-O-C₁₋₆alkyl,
   C₁₋₃ alkylene-heterocyclyl, and
   C₁₋₆ alkylene-C₃₋₇cycloalkyl, or
R³⁰¹ and R³⁰² form together with the nitrogen atom to which they are attached a 5 to 6-membered ring which may additionally contain one oxygen atom in the ring and which ring is unsubstituted or substituted by one or more substituents selected from OH, C₁₋₆alkyl, O-C₁₋₆alkyl, C₀₋₃alkylene-C₃₋₅cycloalkyl, C₁₋₆alkylene-O-C₁₋₆alkyl or (CH₂)₀₋₃-phenyl;
A³⁰⁰ is -NH-,
   -C₁₋₆alkylene,
   -C₂₋₆alkenylene,
   -C₂₋₆alkinylene or
   a bond,
   wherein alkylene, alkenylene and alkinylene are unsubstituted or substituted with one ore more R³⁰⁷;
R³⁰⁷ is independently selected from
   H,
   C₁₋₆alkyl,
   OR³¹⁴,
   NR^{315a}R^{315b},
   halogen,
   phenyl and
   heteroaryl,
   wherein phenyl and heteroaryl are unsubstituted or substituted by 1 to 3 R^{304a};
X³⁰⁰ is H,
   CN,
   phenyl,
   phenyl which is fused with a saturated heterocyclic 6-membered ring, wherein the heterocyclic ring may contain 1 or 2 heteroatoms selected from O and N and wherein the heterocyclic ring may further be optionally substituted by an oxo group,
   4 to 8-membered saturated or unsaturated heterocyclyl containing 1, 2, 3 or 4 heteroatoms independently selected from N, O and S,
   5- to 6-membered heteroaryl containing 1, 2, 3, or 4 heteroatoms independently selected from N, O and S, or
   -C(O)-R³⁰⁶,
   C₃₋₈cycloalkyl, unsubstituted or substituted with one or more halogen atoms,
   -OR³¹⁴,
   halogen or
   NR^{315a}R^{315b},
   wherein each phenyl, heterocyclyl and heteroaryl is optionally substituted by 1 to 3 R^{304a} and/or 1 R^{304b} and/or 1 R³⁰⁵;
R^{304a} is halogen,
   CN,
   C₁₋₆alkyl, optionally substituted with one or more halogen atoms,
   O-C₁₋₆alkyl, optionally substituted with one or more halogen atoms, or
   OH;
R^{304b} is C(O)NH₂,
   C(O)NH-C₁₋₆alkyl,
   C(O)N-(C₁₋₆alkyl)₂,
   SO₂-C₁₋₆alkyl,
   C(O)NH-SO₂-C₁₋₆alkyl,
   oxo, whereby the ring is at least partially saturated,
   NH₂,
   NH-C₁₋₆alkyl,
   N-(C₁₋₆alkyl)₂,
   NH-SO₂-CH₃, or
   NH-SO₂-CF₃;
R³⁰⁵ is 5 to 6-membered saturated or unsaturated heterocyclyl containing 1 to 3 heteroatoms independently selected from N, O and S or
   5 to 6-membered heteroaryl containing 1 to 3 heteroatoms independently selected from N, O and S,
   wherein the heterocyclyl and the heteroaryl are optionally substituted by 1 or 2 R^{304a};
R³⁰⁶ is H,
   C₁₋₆alkyl, optionally substituted with one or more halogen atoms, phenyl or
   4 to 8-membered saturated or unsaturated heterocyclyl containing 1 to 3 heteroatoms selected from N, O and S,
   wherein each phenyl and heterocyclyl is optionally substituted by 1 to 3 R^{304a} and/or 1 R³⁰⁵,
   OH,
   O-C₁₋₆alkyl, wherein alkyl is unsubstituted or substituted with one or more
   R³¹⁶, or
   NR^{316a}R^{316b};
R³⁰³ is -(CR³⁰⁸R³⁰⁹)ₙ₃₀₀-T³⁰⁰;
R³⁰⁸ and R³⁰⁹ are independently from each other selected from
   H,
   OH,
   halogen,
   C₁₋₆alkyl, and
   O-C₁₋₆ alkyl;
n300 is 1 to 6;
T³⁰⁰ is 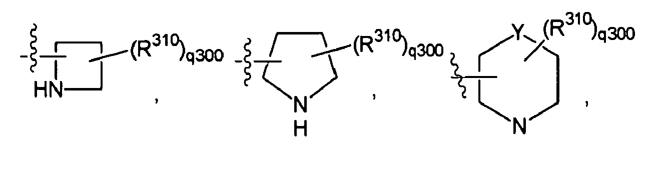 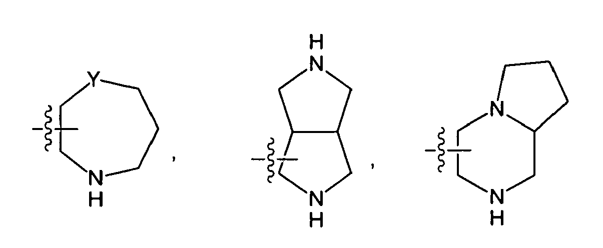 or NR³¹²R³¹³;
R³¹⁰ is H,
   NH₂,
   OH,
   C₁₋₆alkyl,
   halogen,
   NH(C₁₋₆alkyl),
   N(C₁₋₆alkyl)₂,
   phenyl or
   heteroaryl,
   wherein phenyl and heteroaryl are optionally substituted by 1 to 3 R^{304a};
q300 is 1 or 2
Y³⁰⁰ is CH₂, NR³¹¹ or O;
R³¹¹ is H,
   C₁₋₆alkyl or
   (CH₂)₀₋₆-C₃₋₇cycloalkyl;
R³¹² and R³¹³ are independently from each other selected from
   H,
   C₁₋₆ alkyl,
   (CH₂)₀₋₂-C₃₋₇cycloalkyl and
   C₁₋₆alkylene-O-C₁₋₆alkyl;
   wherein C₁₋₆alkyl, C₁₋₆alkylene and C₃₋₇cycloalkyl are optionally substituted by 1 to 3 R^{304a};
R³¹⁴ is H or
   C₁₋₆alkyl, unsubstituted or substituted with one or more substituents selected from halogen,
   phenyl or heteroaryl,
   wherein phenyl and heteroaryl are optionally substituted by 1 to 3 R^{304a};
R^{315a} and R^{315b} are independently from each other selected from
   H,
   C₁₋₆alkyl, unsubstituted or substituted with one or more substituents selected from halogen, OH, O(C₁₋₆alkyl), NH₂, NH(C₁₋₆alkyl) and N(C₁₋₆ alkyl)₂,
   phenyl or heteroaryl,
   wherein phenyl and heteroaryl are optionally substituted by 1 to 3 R^{304a} and
   C(O)C₁₋₆alkyl;
R³¹⁶, R^{316a} and R^{316b} are independently from each other selected from
   H,
   C₁₋₆alkyl, unsubstituted or substituted with halogen, OH, O(C₁₋₆alkyl),
   NH₂, NH(C₁₋₆alkyl), N(C₁₋₆ alkyl)₂,
   C₀₋₃alkylene-C₃₋₅cycloalkyl,
   phenyl and
   heteroaryl,
   wherein phenyl and heteroaryl are unsubstituted or substituted by 1 to 3 R^{304a}; 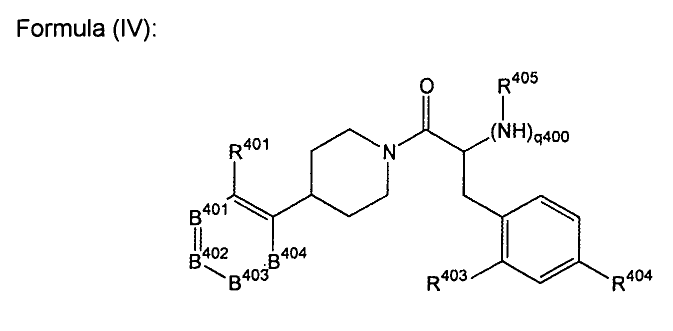 and the enantiomers, diastereomers, tautomers, solvates and pharmaceutically acceptable salts thereof,
   wherein
R⁴⁰¹ is -N(R⁴¹⁰)-(C(R⁴⁰⁶)₂)ₘ₄₀₀-T⁴⁰⁰
   -(C(R⁴⁰⁶)₂)ₗ₄₀₀-T⁴⁰⁰, or
   -O-(C(R⁴⁰⁶)₂)ₘ₄₀₀-T⁴⁰⁰;
R⁴⁰⁶ is independently selected from
   H,
   F,
   OH,
   OCH₃,
   C₁₋₆-alkyl, optionally substituted with 1 to 3 substituents selected from halogen, CN, OH and OCH₃, and
   C₃₋₆-cycloalkyl, optionally substituted with 1 to 3 substituents selected from halogen, CN, OH and OCH₃;
T⁴⁰⁰ is NR⁴⁰⁷R⁴⁰⁸,
   morpholine, 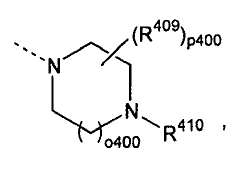 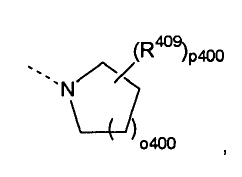 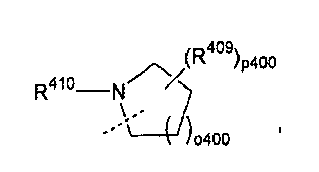 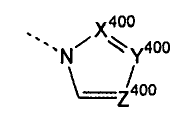 or 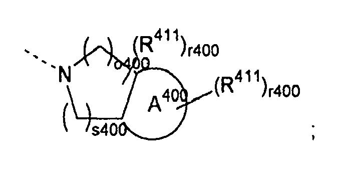
R⁴⁰⁷ and R⁴⁰⁸ are independently from each other selected from
   H,
   C₁₋₆-alkyl,
   C₂₋₆-alkenyl,
   C₂₋₆-alkinyl, and
   C₂₋₆-alkylene-O-C₁₋₆-alkyl,
   wherein each alkyl, alkenyl and alkinyl is optionally substituted by one or more halogen atoms, CN or OH;
R⁴⁰⁹ is independently selected from
   halogen,
   CN,
   OH,
   C₁₋₆-alkyl optionally substituted with 1 to 3 substituents selected from halogen, CN and OH,
   O-C₁₋₆-alkyl optionally substituted with 1 to 3 substituents selected from halogen, CN and OH, and
   C₁₋₆-alkylene-O-C₁₋₆-alkyl optionally substituted with 1 to 3 substituents selected from halogen, CN and OH;
R⁴¹⁰ is H, or
   C₁-C₆-alkyl;
R⁴¹¹ is independently selected from
   halogen,
   CN,
   OH,
   C₁₋₆-alkyl optionally substituted with 1 to 3 substituents selected from halogen, CN and OH,
   O-C₁₋₆-alkyl optionally substituted with 1 to 3 substituents selected from halogen, CN and OH,
   C₁₋₆-alkylene-O-C₁₋₆-alkyl optionally substituted with 1 to 3 substituents selected from halogen, CN and OH,
   -NH₂,
   -NH(C₁₋₆-alkyl), and
   -N(C₁₋₆-alkyl)₂;
X⁴⁰⁰ is CH or N;
Y⁴⁰⁰ is CH or N;
Z⁴⁰⁰ is CH or N;
A⁴⁰⁰ is a 3-7-membered saturated, unsaturated or aromatic ring containing 0-2 nitrogen atoms;
B⁴⁰¹ to B⁴⁰⁴ are independently selected from CR⁴⁰² and N;
R⁴⁰² is independently selected from
   H,
   F,
   Cl,
   CH₃,
   OCH₃, and
   CF₃;
R⁴⁰³ is H,
   Cl,
   F, or
   CH₃;
R⁴⁰⁴ is Cl or F;
R⁴⁰⁵ is 4 to 7-membered heterocyclyl containing 1 or 2 heteroatoms independently selected from the group consisting of NR⁴¹², O and S; or
   4 to 7-membered heteroaryl containing 1 to 4 heteroatoms independently selected from the group consisting of N, O and S;
   wherein each heterocyclyl may be unsubstituted or substituted by 1 or 2 substituents selected from the group consisting of oxo group, CN, C₁₋₆-alkyl, C₃₋₆-cycloalkyl, halogen and OH, and
   each heteroaryl may be unsubstituted or substituted by 1 or 2 substituents selected from the group consisting of C₁₋₆-alkyl, C₃₋₆-cycloalkyl, O-C₁₋₆-alkyl, halogen and CN;
R⁴¹² is hydrogen,
   C₁₋₆-alkyl,
   C₃₋₆-cycloalkyl and C(O)C₁₋₆-alkyl;
l400 is 0, 1, 2, 3, or 4;
m400 is 0, 1, 2, 3, or 4;
o400 is 0, 1, or 2;
p400 is 0, 1, 2, 3, or 4;
q400 is 0, or 1;
r400 is 0, 1, 2, 3, or 4 and
s400 is 1, or 2; 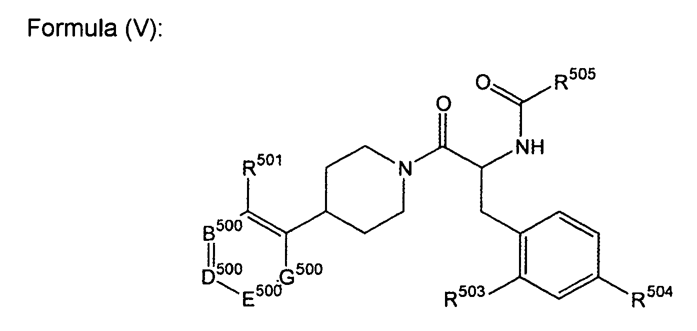 and the enantiomers, diastereomers, tautomers, solvates and pharmaceutically acceptable salts thereof,
   wherein
R⁵⁰¹ is -N(R⁵¹⁰)-(C(R⁵⁰⁶)₂)ₘ₅₀₀-T⁵⁰⁰
   -(C(R⁵⁰⁶)₂)ₗ₅₀₀-T⁵⁰⁰, or
   -O-(C(R⁵⁰⁶)₂)ₘ₅₀₀-T⁵⁰⁰;
R⁵⁰⁶ is independently selected from
   H,
   F,
   OH,
   OCH₃,
   C₁₋₆-alkyl, optionally substituted with 1 to 3 substituents selected from halogen, CN, OH and OCH₃, and
   C₃₋₆-cycloalkyl, optionally substituted with 1 to 3 substituents selected from halogen, CN, OH and OCH₃;
T⁵⁰⁰ is NR⁵⁰⁷R⁵⁰⁸,
   morpholine, 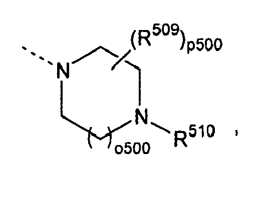 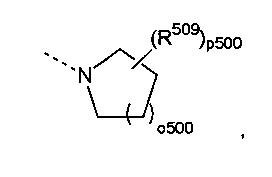 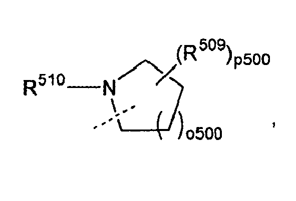 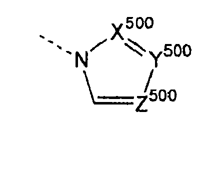 or 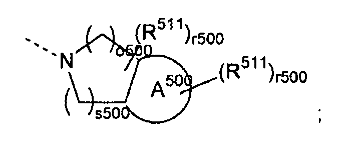
R⁵⁰⁷ and R⁵⁰⁸ are independently from each other selected from
   H,
   C₁₋₆-alkyl,
   C₂₋₆-alkenyl,
   C₂₋₆-alkinyl, and
   C₂₋₆-alkylene-O-C₁₋₆-alkyl,
   wherein each alkyl, alkenyl and alkinyl is optionally substituted by one or more halogen atoms, CN or OH;
R⁵⁰⁹ is independently selected from
   halogen,
   CN,
   OH,
   C₁₋₆-alkyl optionally substituted with 1 to 3 substituents selected from halogen, CN and OH, and
   O-C₁₋₆-alkyl optionally substituted with 1 to 3 substituents selected from halogen, CN and OH,
   C₁₋₆-alkylene-O-C₁₋₆-alkyl optionally substituted with 1 to 3 substituents selected from halogen, CN and OH;
R⁵¹⁰ is H, or
   C₁-C₆-alkyl;
R⁵¹¹ is independently selected from
   halogen,
   CN,
   OH,
   C₁₋₆-alkyl optionally substituted with 1 to 3 substituents selected from halogen, CN and OH,
   O-C₁₋₆-alkyl optionally substituted with 1 to 3 substituents selected from halogen, CN and OH,
   C₁₋₆-alkylene-O-C₁₋₆-alkyl optionally substituted with 1 to 3 substituents selected from halogen, CN and OH,
   -NH₂,
   -NH(C₁₋₆-alkyl), and
   -N(C₁₋₆-alkyl)₂;
X⁵⁰⁰ is CH or N;
Y⁵⁰⁰ is CH or N;
Z⁵⁰⁰ is CH or N;
A⁵⁰⁰ is a 3-7-membered saturated, unsaturated or aromatic ring containing 0-2 nitrogen atoms;
B⁵⁰⁰ is CR⁵⁰² or N;
G⁵⁰⁰ is CR⁵⁰² or N;
D⁵⁰⁰ is CR⁵⁰² or N;
E⁵⁰⁰ is CR⁵⁰² or N;
   with the proviso that one or two of the variables B⁵⁰⁰, G⁵⁰⁰, D⁵⁰⁰ and E⁵⁰⁰ must be N_{;}
   R⁵⁰² is independently selected from
   H,
   F,
   Cl,
   CH₃,
   OCH₃, and
   CF₃;
R⁵⁰³ is H,
   Cl,
   F, or
   CH₃;
R⁵⁰⁴ is Cl or F;
R⁵⁰⁵ is 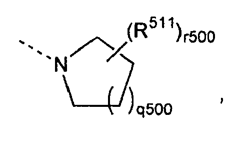 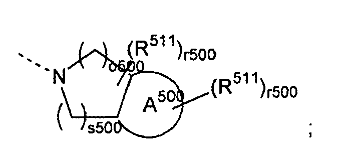 morpholine, optionally substituted by 1 to 3, same or different substituents R⁵¹⁴, or
   NR⁵¹²R⁵¹³;
R⁵¹² and R⁵¹³ are independently from each other selected from
   C₁₋₆-alkyl,
   C₂₋₆-alkenyl,
   C₂₋₆-alkinyl,
   C₂₋₆-alkylene-O-C₁₋₆-alkyl, and
   C₂₋₆-alkylene-N-(C₁₋₆-alkyl)₂;
R⁵¹⁴ is C₁₋₆-alkyl,
   C₁₋₆-alkylene-O-C₁₋₆-alkyl,
   C₁₋₆-alkylene-OH,
   C₁₋₆-alkylene-NH₂,
   C₁₋₆-alkylene-NH-C₁₋₆-alkyl, or
   C₁₋₆-alkylene-N(C₁₋₆-alkyl)₂;
l500 is 0, 1, 2, 3, or 4;
m500 is 0, 1, 2, 3, or 4;
o500 is 0, 1, or 2;
p500 is 0, 1, 2, 3, or 4;
q500 is 0, 1, 2, or 3;
r500 is 0, 1, 2, 3, or 4 and
s500 is 1, or 2.

Further preferred melanocortin-4 receptor antagonists are selected from the group consisting of compounds of the general structural formulae (VI) to (XIX): 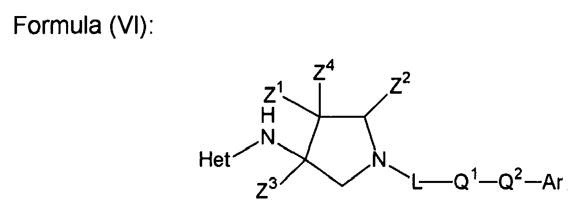 wherein Het, Ar, L, Q¹, Q², and Z¹ to Z⁴ are defined as in claim 1 of WO 2007/114323; 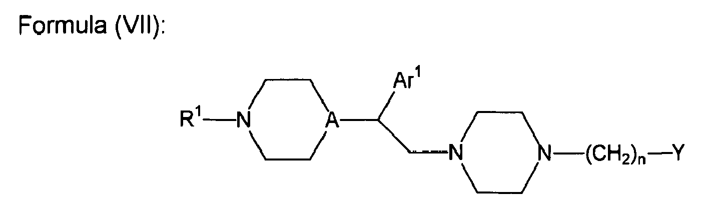 wherein Ar¹, A, Y, R¹ und n are defined as in claim 1 of WO 2003/053927; 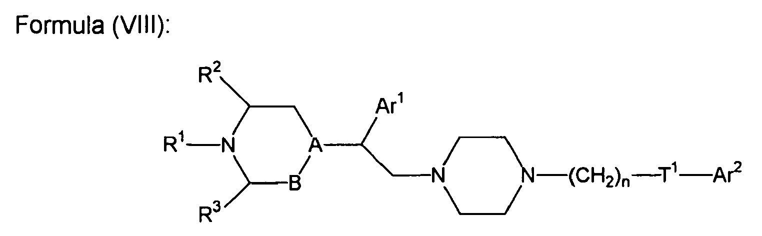 wherein Ar¹, Ar², A, B, T¹, R¹ to R³ and n are defined as in claim 2 of WO 2002/00259; 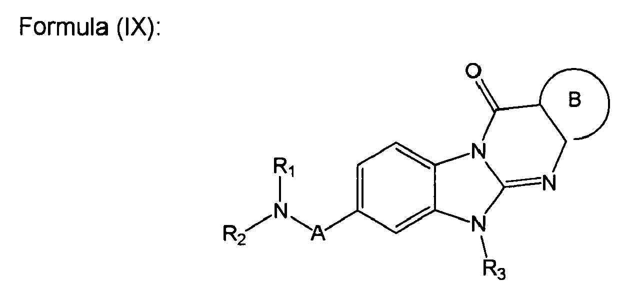 wherein A, B and R₁ to R₃ are defined as in claim 1 of WO 2006/010811; 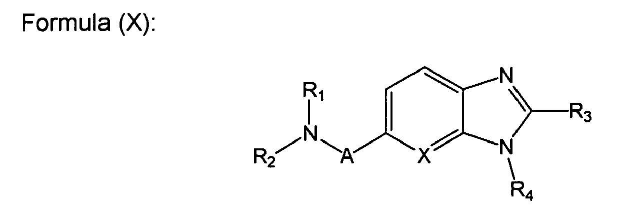 wherein A, X and R₁ to R₄ are defined as in claim 1 of WO 2005/056533; 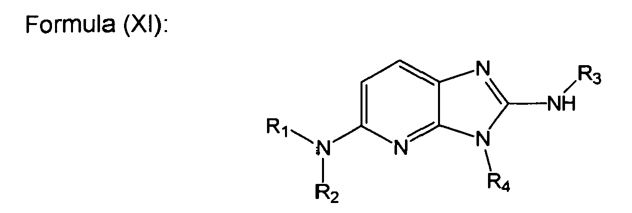 wherein R₁ to R₄ are defined as in claim 1 of WO 2004/089951; 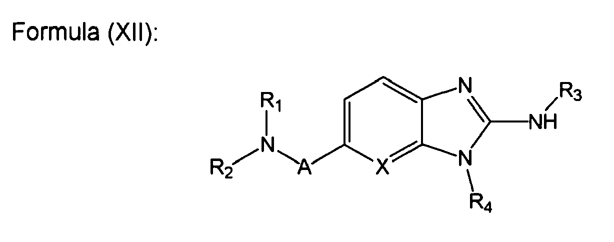 wherein A, X and R₁ to R₄ are defined as in claim 1 of WO 2004/075823; 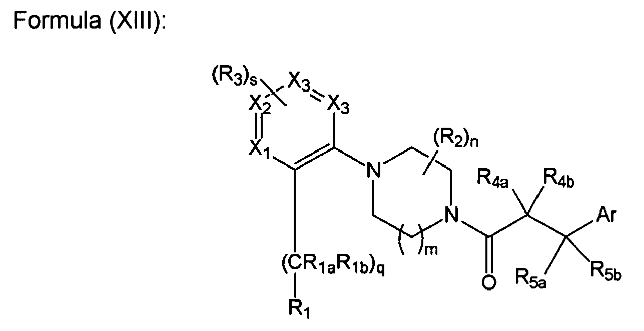 wherein Ar, X₁ to X₄, R₁, R₁ₐ, R_{1b}, R₂, R₃, R₄ₐ, R_{4b}, R₅ₐ, R_{5b}, m, n, q and s are defined as in claim 1 of WO 2005/042516; 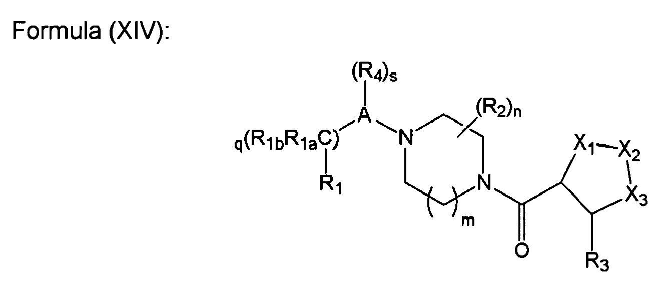 wherein X₁ to X₃, R₁ to R₄, R₁ₐ, R_{1b}, m, n, q and s are defined as in claim 1 of WO 2005/040109; 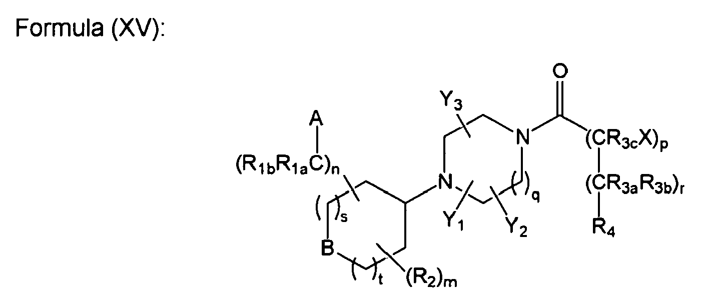 wherein A, B, X, Y₁ to Y₃, R₁ₐ, R_{1b}, R₂, R₃ₐ, R_{3b}, R_{3c}, R₄, m, n, p, q, r, s and t are defined as in claim 1 of WO 2004/058735; 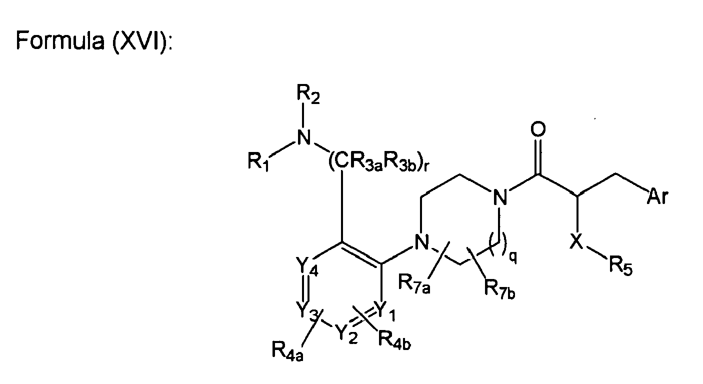 wherein Ar, X, Y₁ to Y₄, R₁, R₂, R₃ₐ, R_{3b}, R₄ₐ, R_{4b}, R₅, R₇ₐ, R_{7b}, q and r are defined as in claim 1 of WO 2003/094918; 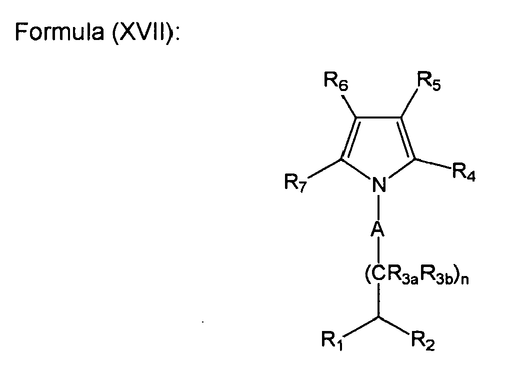 wherein A, R₁, R₂, R₃ₐ, R_{3b}, R₄ to R₇ and n are defined as in claim 1 of WO 2003/068738; 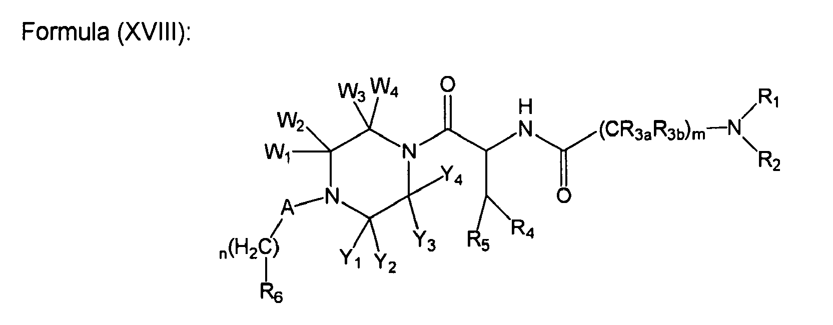 wherein A, X, Y₁ to Y₄, W₁ to W₄, R₁, R₂, R₃ₐ, R_{3b}, R₄, R₅, R₆, m and n are defined as in claim 1 of WO 2003/031410; and 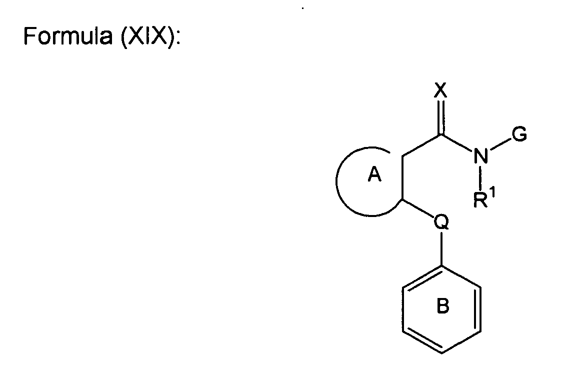 wherein A, B, G, Q, X and R₁ are defined as in claim 1 of WO 2004/050610.

Equally preferred melanocortin-4 receptor antagonists are those mentioned in WO 2002/062766 as follows:
2-[2-(4-benzyloxy-benzylsulfanyl)-phenyl]-1,4,5,6-tetrahydro-pyrimidine;
2-[2-(2-iodo-benzylsulfanyl)-phenyl]-1,4,5,6-tetrahydro-pyrimidine;
2-[2-(2-methoxy-5-nitro-benzylsulfanyl)-phenyl]-1,4,5,6-tetrahydro-pyrimidine;
2-[2-(naphthalen-1-ylmethylsulfanyl)-phenyl]-1,4,5,6-tetrahydro-pyrimidine;
2-[2-(3-chloro-benrylsulfanyl)-phenyl]-1,4,5,6-tetrahydro-pyrimidine;
2-[2-(2,5-dimethoxy-benzylsulfanyl)-phenyl]-1,4,5,6-tetrahydro-pyrimidine;
2-[2-(3-bromo-benzylsulfanyl)-phenyl]-1,4,5,6-tetrahydro-pyrimidine;
2-[2-(2-iodo-benzylsulfanyl)-phenyl]-4,5-dihydro-1H-imidazole;
2-[2-(2-methoxy-5-nitro-benzylsulfanyl)-phenyl]4,5-dihydro-1H-imidazole;
2-[2-(2-methoxy-5-nitro-benzyloxy)-phenyl]-1,4,5,6-tetrahydropyrimidine;
2-[2-(2-bromo-benzylsulfanyl)-phenyl]-1,4,5,6-tetrahydro-pyrimidine;
2-[2-(3-iodo-benzylsulfanyl)-phenyl]-1,4,5,6-tetrahydro-pyrimidine;
2-[2-(2-methoxy-5-nitro-benzylsulfanyl)-phenyl]-3a,4,5,6,7,7a-hexahydro-1H-benzoimidazole;
2-{2-[2-(2-methoxy-naphthalen-1-yl)-ethyl]-phenyl}-1,4,5,6-tetrahydropyrimidine;
2-[2-(5-bromo-2-methoxy-benzylsulfanyl)-phenyl]-1,4,5,6,-tetrahydropyrimidine;
2-{2-[2-(2-methyl-naphthalen-1-yl)-ethyl)-phenyl}-1,4,5,6-tetrahydropyrimidine;
2-{2-[2-(2,3-dihydro-benzo[1,4]dioxin-5-yl)-ethyl]-phenyl}-1,4,5,6-tetrahydropyrimidine;
2-[2-(2-methoxy-napthalen-1-ylmethylsulfanyl)-phenyl]-1,4,5,6-tetrahydropyrimidine;
2-(2-Benzylsulfanyl-phenyl)-1,4,5,6-tetrahydro-pyrimidine;
2-(2-Pentadecylsulfanyl-phenyl)-1,4,5,6-tetrahydro-pyrimidine;
2-(2-Cyclohexylmethylsulfanyl-phenyl)-1,4,5,6-tetrahydro-pyrimidine;
2-[2-(2-Methyl-benzylsulfanyl)-phenyl]-1,4,5,6-tetrahydro-pyrimidine;
2-[2-(3-Nitro-benzylsulfanyl)-phenyl]-1,4,5,6-tetrahydro-pyrmudine;
2-[2-(3,5-Dimethoxy-benzylsulfanyl)-phenyl]-1,4,5,6-tetrahydro-pyrimidine;
2-[2-(4-Fluoro-benzylsulfanyl)-phenyl]-1,4,5,6-tetrahydro-pyrimidine;
2-[2-(2-Chloro-benzylsulfanyl)-phenyl]-1,4,5,6-tetrahydro-pyrimidine;
2-[2-(2-Fluoro-benzylsulfanyl)-phenyl]-1,4,5,6-tetrahydro-pyrimidine;
2-[2-(2,4-Bis-trifluoromethyl-benzylsulfanyl)-phenyl]-1,4,5,6-tetrahydro-pyrimidine;
2-[2-(3-Methoxy-benzylsulfanyl)-phenyl]-1,4,5,6-tetrahydro-pyrimidine;
2-[2-(3,5-Bis-trifluoromethyl-benzylsulfanyl)-phenyl]-1,4,5,6-tetraydro-pyrimidine;
2-[2-(2-Methoxy-5-nitro-benzyloxy)-phenyl]-1,4,5,6-tetrahydro-pyrimidine;
2-[2-(2-Chloro-6-fluoro-benzylsulfanyl)-phenyl]-4,5-dihydro-1H-imidazole;
2-(2-Benzylsulfanyl-phenyl)-4,5-dihydro-1H-imidazole;
2-[2-(2,6-Difluoro-benzylsulfanyl)-phenyl]-1,4,5,6-tetrahydro-pyrimidine;
2-[2-(Naphthalen-1-ylmethoxy)-phenyl]-1,4,5,6-tetrahydro-pyrimidine;
2-[2-(2-Methyl-naphthalen-1-ylmethylfanyl)-phenyl]-1,4,5,6-tetrahydro-pyrimidine;
1-{2-[2-(2-Chloro-6-fluoro-benzylsulfanyl)-phenyl]-5,6-dihydro-4H-pyrimidin-1-yl}-ethanone;
2-[2-(2-Chloro-6-fluoro-benzylsulfanyl)-phenyl]-3a,4,5,6,7,7a-hexahydro-1H-benzoimidazole;
2-[2-(2-Iodo-benzylsulfanyl)-phenyl]-3a,4,5,6,7,7a-hexahydro-1H-benzoimidazole;
2-[2-(2,5-Dimethyl-benzylsulfanyl)-phenyl]-1,4,5,6-tetrahydro-pyrimidine;
4-[2-(1,4,5,6-Tetrahydro-pyrimidin-2-yl)-phenylsulfanylmethyl]-quinoline;
2-[2-(2-Methoxy-5-nitro-benzylsulfanyl)-pyridin-3-yl]-1,4,5,6-tetrahydro-pyrimidine;
2-[2-(2-Methoxy-benzylsulfanyl)-phenyl]-1,4,5,6-tetrahydro-pyrimidine;
2-[2-(2-Cyclopentyloxy-benzylsulfanyl)-phenyl]-1,4,5,6-tetrahydro-pyrimidine;
2-[2-(2,3-Dihydro-benzo[1,4]dioxin-5-ylmethylsulfanyl)-phenyl]-1,4,5,6-tetrahydro-pyrimidine;
2-[2-(6-Methoxy-2,3-dihydro-benzo[1,4]dioxin-5-ylmethylsulfanyl)-phenyl]-1,4,5,6-tetrahydro-pyrimidine;
2-[2-(5-fluoro-2-methoxy-benzylsulfanyl)-phenyl]-4,5-dihydro-1H-imidazole;
1-Methyl-2-[2-(naphthalen-1-ylmethylsulfanyl)-phenyl]-1,4,5,6-tetrahydro-pyrimidine;
2-[2-(5-Bromo-2-methoxy-benzylsulfanyl)-phenyl]-4,5-dihydro-1 H-imidazole;
2-[2-(5-Bromo-2-methoxy-benzyloxy)-phenyl]-1,4,5,6-tetrahydro-pyrimidine;
2-[2-(Naphthalen-1-yloxymethyl)-phenyl]-1,4,5,6-tetrahydro-pyrimidine;
2-[2-(5-Bromo-2-methoxy-benzylsulfanyl)-phenyl]-5,5-dimethyl-1,4,5,6-tetrahydro-pyrimidine;
2-[2-(5-Bromo-2-methoxy-benzylsulfanyl)-phenyl]-5,5-dimethyl-4,5-dihydro-1H-imidazole;
2-[2-(2,6-Dimethoxy-benzylsulfanyl)-phenyl]-1,4,5,6-tetrahydro-pyrimidine;
2-[2-(2-Bromo-6-methoxy-benzylsulfanyl)-phenyl]-1,4,5,6-tetrahydro-pyrimidine;
2-[5-Bromo-2-(5-bromo-2-methoxy-benzylsulfanyl)-phenyl]-4,5-dihydro-1H-imidazole;
2-[5-Bromo-2-(5-bromo-2-methoxy-benzylsulfanyl)-phenyl]-1,4,5,6-tetrahydro-pyrimidine;
2-[4-Bromo-2-(5-bromo-2-methoxy-benzylsulfanyl)-phenyl]-1,4,5,6-tetrahydro-pyrimidine;
2-[2-(2-Bromo-5-methoxy-benzylsulfanyl)-phenyl]-1,4,5,6-tetrahydro-pyrimidine;
2-[2-(5-Bromo-2-methoxy-benzylsulfanyl)-5-methyl-phenyl]-1,4,5,6-tetrahydro-pyrimidine;
2-[2-(Biphenyl-3-ylmethylsulfanyl)-pheoyl]-1,4,5,6-tetrahydro-pyrimidine;
2-[2-(5-Chloro-2-methoxy-benzylsulfanyl)-phenyl]-1,4,5,6-tetrahydro-pyrimidine;
2-[2-(2-Methoxy-5-thiophen-3-yl-benzylsulfanyl)-phenyl]-1,4,5,6-tetrahydro-pyrimidine;
2-[2-(Biphenyl-2-ylmethylsulfanyl)-phenyl]-1,4,5,6-tetrahydro-pyrimidine;
2-[2-(5-Iodo-2-methoxy-benzylsulfanyl)-phenyl]-1,4,5,6-tetrahydro-pyrimidine;
2-[2-(5-Bromo-2-methoxy-benzylsulfanyl)-5-fluoro-phenyl]-1,4,5,6-tetrahydro-pyrimidine;
2-[2-(5-Bromo-2-methoxy-benzylsulfanyl)-3-fluoro-phenyl]-1,4,5,6-tetrahydro-pyrimidine;
2-[2-(4,4'-Dimethoxy-biphenyl-3-ylmethylsulfanyl)-phenyl]-1,4,5,6-tetrahydro-pyrimidine;
2-[2-(9H-Fluoren-9-ylsulfanyl)-phenyl]-1,4,5,6-tetrahydro-pyrimidine;
2-[2-(3'-Chloro-4'-fluoro-4-methoxy-biphenyl-3-ylmethylsulfanyl)-phenyl]-1,4,5,6-tetrahydro-pyrimidine;
2-[2-(1-Naphthalen-1-yl-ethylsulfanyl)-phenyl]-1,4,5,6-tetrahydro-pyrimidine;
2-[2-(5-Bromo-2-methoxy-benzylsulfanyl)-5-fluore-phenyl]-4,5-dihydro-1H-imidazole;
2-(2-Benzhydrylsulfanyl-phenyl)-1,4,5,6-tetrahydro-pyrimidine;
2-[2-(2'-Fluoro-4"-methoxy-[1,1';4',1"]terphenyl-3"-ylmethylsulfanyl)-phenyl]-1,4,5,6-tetrahydro-pyrimidine;
2-(5-Bromo-2-methoxy-benzylsulfanyl)-benzamidine;
2-[4-(Naphthalen-1-ylmethylsulfanyl)-phenyl]-1,4,5,6-tetrahydro-pyrimidine;
2-[2-(5-Ethynyl-2-methoxy-benzylsulfanyl)-phenyl]-1,4,5,6-tetrahydro-pyrimidine;
2-[2-(5-Bromo-2-methoxy-benzylsulfanyl)-benzyl]-1,4,5,6-tetrahydro-pyrimidine;
2-[2-(5-Bromo-2-cyclopentyloxy-benzylsulfanyl)-phenyl]-1,4,5,6-tetrahydro-pyrimidine;
2-[2-(5-Bromo-2-ethoxy-benzylsulfanyl)-phenyl]-1,4,5,6-tetrahydro-pyrimidine;
2-[2-(5-Bromo-2-propoxy-benzylsulfanyl)-phenyl]-1,4,5,6-tetrahydro-pyrimidine;
[2-(5-Bromo-2-methoxy-benzylsulfanyl)-benzyl]-diethyl-amine;
1-[2-(5-Bromo-2-methoxy-benzylsulfanyl)-benzyl]-piperazine;
C-{4-[3-(5-Bromo-2-methoxy-benzylsulfanyl)-quinoxalin-2-yl]-morpholin-2-yl}-methylamine;
2-[2-(2-Methoxy-5-methyl-benzylsulfanyl)-phenyl]-1,4,5,6-tetrahydro-pyrimidine;
2-[2-(5-Bromo-2-methoxy-benzyloxymethyl)-phenyl]-1,4,5,6-tetrahydro-pyrimidine;
[2-(5-Bromo-2-methoxy-benzylsulfenyl)-benzyl]-dimethyl-amine;
2-[2-(5-Bromo-2-isopropoxy-benzylsulfanyl)-phenyl]-1,4,5,6-tetrahydro-pyrimidine;
2-[2-(2-Ethoxy-naphthalen-1-ylmethylsulfanyl)-phenyl]-1,4,5,6-tetrahydro-pyrimidine;
2-[2-(2-Propoxy-naphthalen-1-ylmethylsulfanyl)-phenyl]-1,4,5,6-tetrahydro-pyrimidine;
4-Methoxy-3-[2-(1,4,5,6-tetrahydro-pyrimidin-2-yl)-phenylsulfanylmethyl]-benzonitrile;
1-{4-Methoxy-3-[2-(1,4,5,6-tetrahydro-pyrimidin-2-yl)-phenylsulfanylmethyl]-phenyl}-ethanone;
2-[2-(Naphthalen-1-ylsulfanylmethyl)-phenyl]-1,4,5,6-tetrahydro-pyrimidine;
1-[2-(5-Bromo-2-methoxy-benzylsulfanyl)-benzyl]-piperidine;
C-{4-[2-(2-Methoxy-naphthalen-1-ylmethylsulfanyl)-benzyl]-morpholin-2-yl}-methylamine;
1-[2-(5-Bromo-2-methoxy-benzylsulfanyl)-benzyl]-pyrrolidin-3-ylamine;
1-[2-(2-Methoxy-naphthalen-1-ylmethylsulfanyl)-benzyl]-pyrrolidin-3-ylamine;
3-[2-(5-Bromo-2-methoxy-benzylsulfanyl)-3-fluoro-phenyl]-1,5,6,7,8,8a-hexahydro-imidazo[1,5-a]pyridine;
3-[2-(5-Bromo-2-methoxy-benzylsulfanyl)-3-fluoro-phenyl]-5,6,7,7a-tetrahydro-1H-pyrrolo[1,2-c]imidazole;
2-[2-(Benzo[b]thiophen-3-ylmethylsulfanyl}-phenyl]-1,4,5,6-tetrahydro-pyrimidine;
2-[3-Fluoro-2-(naphthalen-1-ylmethylsulfanyl)-phenyl]-1,4,5,6-tetrahydro-pyrimidine;
2-(Naphthalen-1-ylmethylsulfanyl)-3-(1,4,5,6-tetrahydro-pyrimidin-2-yl)-phenylamine;
2-[2-(5-Bromo-2-methoxy-benzylsulfanyl)-3-chloro-phenyl]-1,4,5,6-tetrahydro-pyrimidine;
2-[2-(2-Methoxy-phenylsulfanylmethyl)-phenyl]-1,4,5,6-tetrahydro-pyrimidine;
1-{2-[2-(5-Bromo-2-methoxy-benzylsulfanyl)-phenyl]-5,6-dihydro-4H-pyrimidin-1-yl}-3-methyl-butan-1-one;
1-{2-[2-(5-Bromo-2-methoxy-benzylsulfanyl)-phenyl]-5,6-dihydro-4H-pyrimidin-1-yl}-2-phenyl-ethanone;
2-[3-(5-Bromo-2-methoxy-benzylsulfanyl)-pyridin-2-yl]-1,4,5,6-tetrahydro-pyrimidine;
N-[2-(5-Bromo-2-methoxy-benzylsulfanyl)-phenyl]-guanidine;
2-[2-(2-Isopropoxy-naphthalen-1-ylmethylsulfanyl)-phenyl]-1,4,5,6-tetrahydro-pyrimidine;
2-[2-(2-Cyclopentyloxy-naphthalen-1-ylmethylsulfanyl)-phenyl]-1,4,5,6-tetrahydro-pyrimidine;
(5-Bromo-2-methoxy-benzyl)-[2-(1,4,5,6-tetrahydro-pyrimidin-2-yl)-phenyl]-amine;
2-[2-(5-Bromo-2-methoxy-benzylsulfanylmethyl)-phenyl]-1,4,5,6-tetrahydro-pyrimidine;
2-[2-(2-Methoxy-naphthalen-1-ylsulfanylmethyl)-phenyl]-1,4,5,6-tetrahydro-pyrimidine;
2-[3-(5-Bromo-2-methoxy-benzylsuifanyl)-pyrazin-2-yl]-1,4,5,6-tetrahydro-pyrimidine;
2-[3-Chloro-2-(naphthalen-1-ylsulfanylmethyl)-phenyl]-1,4,5,6-tetrahydro-pyrimidine;
2-[2-(6-Bromo-2-methoxy-naphthalen-1-ylmethylsulfanyl)-phenyl]-1,4,5,6-tetrahydro-pyrimidine;
2-[3-Chloro-2-(2-methoxy-naphthalen-1-ylsulfanyhnethyl)-phenyl]-1,4,5,6-tetrahydro-pyrimidine;
2-[2-(5-Bromo-2-methoxy-phenylsulfanylmethyl)-phenyl]-1,4,5,6-tetrahydro-pyrimidine;
2-[2-(5-Bromo-2-methoxy-phenylsulfanylmethyl)-3-chloro-phenyl]-1,4,5,6-tetrahydro-pyrimidine;
2-[1-(2-Naphthalen-1-yl-ethyl)-1H-pyrrol-2-yl]-1,4,5,6-tetrahydro-pyrimidine;
(5-Bromo-2-methoxy-benzyl)-methyl-[2-(1,4,5,6-tetrahydro-pyrimidin-2-yl)-phenyl]-amine;
2-(5-Bromo-2-methoxy-benzylsulfanyl)-benzylamine;
2-[2-(2-Chloro-phenylsulfanylmethyl)-phenyl]-1,4,5,6-tetrahydro-pyrimidine;
2-[2-(2-Bromo-phenylsulfanylmethyl)-phenyl]-1,4,5,6-tetrahydro-pyrimidine;
2-(2-o-Tolylsulfanylmethyl-phenyl)-1,4,5,6-tetrahydro-pyrimidine;
2-[2-(2,5-Dichloro-phenylsulfanylmethyl)-phenyl]-1,4,5,6-tetrahydro-pyrimidine;
2-(3-Amino-propylamino)-6-(5-bromo-2-methoxy-benzylsulfanyl)-benzonitrile;
2-[2-(5-Bromo-2-methoxy-benzylsulfanyl)-benzyl]-1,4,5,6-tetrahydro-pyrimidine;
[2-(5-Bromo-2-methoxy-benzylsulfanyl)-benzyl]-diethyl-amine;
4-[2-(5-Bromo-2-methoxy-benzylsulfanyl)-benzyl]-morpholine;
3'-(5-Bromo-2-methoxy-benzylsulfanyl)-3,4,5,6-tetrahydro-2H-[1,2']bipyrazinyl;
2-(5-Bromo-2-methoxy-benzylsulfanyl)-3-piperazin-1-yl-6,7-dihydro-quinoxaline;
1-[2-(5-Bromo-2-methoxy-benzylsulfanyl)-benzyl]-piperidine;
C-{4-[2-(2-Methoxy-naphthalen-1-ylmethylsulfanyl)-benzyl]-morpholin-2-yl}-methylamine;
1-[3-(5-Bromo-2-methoxy-benzylsulfanyl)-pyrazin-2-yl]-pyrrolidin-3-ylamine;
1-[3-(5-Bromo-2-methoxy-benzylsulfanyl)-quinoxalin-2-yl]-pyrrolidin-3-ylamine;
1-[2-(2-Methoxy-naphthalen-1-ylmethylsulfanyl)-benzyl]-pyrrolidin-3-ylamine;
C-{4-[3-(5-Bromo-2-methoxy-benzylsulfanyl)-pyrazin-2-yl]-morpholin-3-yl}-methylamine;
1-[3-Fluoro-2-(2-naphthalen-1-yl-ethyl)-benzyl]-piperazine;
1-[2-(5-Bromo-2-methoxy-benzylsulfanyl)-3-chloro-benzyl]-azetidine;
1-[2-(5-Bromo-2-methoxy-benzylsulfanyl)-3-chloro-benzyl]-pyrrolidin-3-ol;
[2-(Naphthalen-1-ylmethylsulfanyl)-phenyl]-carbamic acid 1-aza-bicyclo[2.2.2]oct-3-yl ester;
[2-(2-Methyl-naphthalen-1-ylmethylsulfanyl)-phenyl]-carbamic acid 1-aza-bicyclo[2.2.2]oct-3-yl ester;
[2-(2-Methyl-naphthalen-1-ylmethylsulfanyl)-phenyl]-carbamic acid 2-piperidin-1-yl-ethyl ester;
{1-[2-(5-Bromo-2-methoxy-benzylsulfanyl)-3-chloro-benzyl]-pyrrolidin-2-yl}-methanol;
4-tert-Butyl-N-naphthalen-1-ylmethyl-N-(2-piperidin-1-yl-ethyl)-benzamide;
N,N-Dimethyl-N'-naphthalen-2-ylmethyl-N'-naphthalen-1-ylmethyl-propane-1,3-diamine;
N-(5-Bromo-2-methoxy-benzyl)-N',N'-dimethyl-N-naphthalen-1-ylmethyl-propane-1,3-diamine;
1-Naphthalen-1-ylmethyl-3-phenethyl-1-(2-piperidin-1-yl-ethyl)-thiourea;
3-(4-Dimethylamino-phenyl)-1-(3-dimethylamino-propyl)-1-naphthalen-1-ylmethyl-thiourea;
4-[2-(5-Bromo-2-methoxy-benzylsulfanyl)-3-chloro-benzylamino]-piperidine-1-carboxylic acid ethyl ester;
2-[2-(2-Naphthalen-1-yl-ethyl)-phenyl]-ethylamine;
Naphthalene-2-sulfonic acid (2-dimethylamino-ethyl)-naphthalen-1-ylmethyl-amide;
1-[2-(5-Bromo-2-methoxy-benzylsulfanyl)-3-chloro-benzyl]-2-methoxymethyl-pyrrolidine;
(2-Hexyloxy-phenyl)-carbamic acid 2-piperidin-1-yl-1-piperidin-1-ylmethyl-ethyl ester;
3-[2-(5-Bromo-2-methoxy-benzylsulfanyl)-benzyloxy]-pyrrolidine;
2-[2-(5-Bromo-2-methoxy-benzylsulfanyl)-benzyloxymethyl]-pyrrolidine;
2-[2-(Naphthalen-1-ylsulfanylmethyl)-phenyl]-piperidine;
3-[2-(5-Bromo-2-methoxy-benzylsulfanyl)-benzylamino]-propan-1-ol;
3-[2-(5-Bromo-2-methoxy-benzylsulfanyl)-benzylamino]-3-methyl-butan-1-ol;
1-[2-(5-Bromo-2-methoxy-benzylsulfanyl)-benzyl]-pyrrolidin-3-ol;
{1-[2-(5-Bromo-2-methoxy-benzylsulfanyl)-benzyl]-pyrrolidin-2-yl}-methanol;
{1-[2-(Naphthalen-1-ylsulfanylmethyl)-benzyl]-piperidin-2-yl}-methanol;
2-[2-(Naphthalen-1-ylsulfanylmethyl)-pyrrolidin-1-yl]-ethyl-N-pyrrolidine;
N-pyrrolyl-[1-(2-naphthanlen-1-yl-ethyl)-pyrrolidin-2-ylmethyl]-amine;
1-(2-Naphthalen-1-yl-ethyl)-piperidine-2-carboxylic acid methyl ester;
(3-Bromo-benzyl)-(1-ethyl-pyrrolidin-2-ylmethyl)-naphthalen-1-ylmethyl-amine;
3-[2-(5-Bromo-2-methoxy-benzylsulfanyl)-benzyloxy]-piperidine;
(5-Bromo-2-methoxy-benzyl)-(1-ethyl-pyrrolidin-2-ylmethyl)-naphthalen-1-ylmethyl-amine;
(1-Ethyl-pyrrolidin-2-ylmethyl)-naphthalen-2-ylmethyl-naphthalen-1-ylmethyl-amine;
2-[2-(5-Bromo-2-methoxy-benzylsulfanyl)-benzyloxymethyl]-pyrrolidine;
(3-Bromo-benzyl)-(3-imidazol-1-yl-propyl)-naphthalen-1-ylmethyl-amine;
(3-Imidazol-1-yl-propyl)-naphthalen-2-ylmethyl-naphthalen-1-ylmethyl-amine;
[2-(Naphthalen1-ylmethylsulfanyl)-phenyl]-carbamic acid 2-piperidin-1-yl-1-piperidin-1-ylmethyl-ethyl ester;
[2-(Naphthalen-1-ylmethylsulfanyl)-phenyl]-carbamic acid 2-dimethylamino-ethyt ester;
1-[2-(Naphthalen-1-ylsulfanylmethyl)-benzyl]-piperazine;
[3-(2-Methyl-piperidin-1-yl)-propyl]-[2-(naphthalen-1-ylsulfanylmethyl)-benzyl]-amine;
1-[3-Chloro-2-(naphthalen-1-ylsulfanylmethyl)-benzyl]-piperazine;
N,N-Dimethyl-N'-(2-naphthalen-1-yl-ethyl)-N-naphthalen-1-ylmethyl-ethane-1,2-diamine;
{1-[2-(5-Bromo-2-methoxy-benzylsulfanyl)-3-chloro-benzyl]-piperidin-2-yl}-methanol;
1-[2-(2-Naphthalen-1-yl-ethyl)-benzyl]-piperazine;
[3-(2-Methyl-piperidin-1-yl)-propyl]-[2-(2-naphthalen-1-yl-ethyl)-benzyl]-amine;
1-[3-Fluoro-2-(2-naphthalen-1-yl-ethyl)-benzyl]-piperazine;
{1-[3-Chloro-2-(naphthalen-1-ylsulfanylmethyl)-benzyl]-piperidin-2-yl}-methanol;
{1-[2-(5-Bromo-2-methoxy-benzylsulfanyl)-3-chloro-benzyl]-piperidin-2-yl}-methanol;
{1-[2-(2-Naphthalen-1-yl-ethyl)-benzyl]-piperidin-2-yl}-methanol;
[3-(2-Methyl-piperidin-1-yl)-propyl]-[2-(2-naphthalen-1-yl-ethyl)-benzyl]-amine;
1-[2-(2-Naphthalen-1-yl-ethyl)-benzyl]-pyrrolidin-3-ylamine;
1-Phenyl-3-piperazin-1-yl-5,6,7,8-tetrahydro-isoquinoline-4-carbonitrile;
2-[2-(5-Bromo-2-methoxy-benzylsulfanyl)-phenyl]-6-ethyl-1,4,5,6-tetrahydro-pyrimidine;
2-[2-(4-Methoxy-biphenyl-3-ylmethylsulfanyl)-phenyl]-1,4,5,6-tetrahydro-pyrimidine;
2-[2-(2-Methoxy-5-phenylethynyl-benzylsulfanyl)-phenyl]-1,4,5,6-tetrahydro-pyrimidine;
2-[2-(2-Naphthalen-1-yl-ethyl)-phenyl]-1,4,5,6-tetrahydro-pyrimidine;
2-[3-(2-Methoxy-naphthalen-1-ylsulfanylmethyl)-thiophen-2-yl]-1,4,5,6-tetrahydro-pyrimidine;
2-[2-(2,5-Dimethoxy-phenylsulfanylmethyl)-phenyl]-1,4,5,6-tetrahydro-pyrimidine;
2-[2-(4-Methyl-naphthalen-1-ylmethylsulfanyl)-phenyl]-1,4,5,6-tetrahydro-pyrimidine;
2-[2-(5-Bromo-2-methoxy-benzylsulfanyl)-3-fluoro-phenyl]-4,4-dimethyl-4,5-dihydro-1H-imidazole;
2-[2-(5-Bromo-2-methoxy-benzylsulfanyl)-3-fluoro-phenyl]-5,5-dimethyl-1,4,5,6-tetrahydro-pyrimidine;
2-[3-(Naphthalen-1-ylsulfanylmethyl)-thiophen-2-yl]-1,4,5,6-tetrahydro-pyrimidine;
2-{2-[2-(5-Bromo-2-methoxy-phenyl)-ethyl]-phenyl}-1,4,5,6-tetrahydro-pyrimidine;
2-[3-Chloro-2-(2-naphthalen-1-yl-ethyl)-phenyl]-1,4,5,6-tetrahydro-pyrimidine;
2-{2-[2-(5-Bromo-2-methoxy-phenyl)-ethyl]-3-fluoro-phenyl}-1,4,5,6-tetrahydro-pyrimidine;
2-[2-(5-Bromo-2-methoxy-phenylsulfanylmethyl)-3-fluoro-phenyl]-1,4,5,6-tetrahydro-pyrimidine;
2-[2-(Naphthalen-1-ylsulfanylmethyl)-phenyl]-4,5-dihydro-1H-imidazole;
2-[3-Fluoro-2-(naphthalen-1-ylsulfanylmethyl)-phenyl]-1,4,5,6-tetrahydro-pyrimidine;
2-[3-Bromo-2-(naphthalen-1-ylsulfanylmethyl)-phenyl]-1,4,5,6-tetrahydro-pyrimidine;
2-{2-[2-(5-Bromo-2-methoxy-phenyl)-ethyl]-3-chloro-phenyl}-1,4,5,6-tetrahydro-pyrimidine;
2-[2-(2-Methoxy-5-trifluoromethyl-benzylsulfanyl)-phenyl]-1,4,5,6-tetrahydro-pyrimidine;
2-[4-(Naphthalen-1-ylsulfanylmethyl)-thiophen-3-yl]-1,4,5,6-tetrahydro-pyrimidine;
2-[2-(Naphthalen-1-ylsulfanylmethyl)-thiophen-3-yl]-1,4,5,6-tetrahydro-pyrimidine;
2-{2-[2-(5-Bromo-2-methoxy-phenyl)-ethyl]-3-trifluoromethyl-phenyl}-1,4,5,6-tetrahydro-pyrimidine;
2-[2-(2-Naphthalen-1-yl-ethyl)-3-trifluoromethyl-phenyl]-1,4,5,6-tetrahydro-pyrimidine;
2-[2-(6-Fluoro-naphthalen-1-ylmethylsulfanyl)-phenyl]-1,4,5,6-tetrahydro-pyrimidine;
{1-[2-(5-Bromo-2-methoxy-benzylsulfanyl)-benzyl]-piperidin-2-yl}-methanol;
2-[3-Fluoro-2-(2-naphthalen-1-yl-ethyl)-phenyl]-1,4,5,6-tetrahydro-pyrimidine;
[2-(5-Bromo-2-methoxy-benzylsulfanyl)-3-chloro-benzyl]-[3-(2-methyl-piperidin-1-yl)-propyl]-mine;
1-[2-(5-Bromo-2-methoxy-benzylsulfanyl)-3-chloro-benzyl]-pyrrolidin-3-ylamine;
1-[2-(5-Bromo-2-methoxy-benzylsulfanyl)-3-chloro-benzyl]-piperazine;
5,5-Dimethyl-2-[2-(2-naphthalen-1-yl-ethyl)-phenyl]-4,5-dihydro-1H-imidazole;
2-[3-Fluoro-2-(2-naphthalen-1-yl-ethyl)-phenyl]-5,5-dimethyl-4,5-dihydro-1H-imidazole;
2-[2-(5-Bromo-2-methoxy-benzylsulfanyl)-3,5-difluoro-phenyl]-1,4,5,6-tetrahydro-pyrimidine;
2-[2-(5-Bromo-2-methoxy-benzylsulfanyl)-3,5-difluoro-phenyl]-5,5-dimethyl-4,5-dihydro-1H-imidazole;
3-(2-Naphthalen-1-yl-ethyl)-2-(1,4,5,6-tetrahydro-pyrimidin-2-yl)-phenylamine;
Amino-[2-(2-naphthalen-1-yl-ethyl)-phenyl]-acetonitrile;
1-[2-(2-Naphthalen-1-yl-ethyl)-phenyl]-ethane-1,2-diamine;
2-[2-(5-Bromo-2-methoxy-benzylsulfanyl)-phenyl]-4-methyl-4,5-dihydro-1H-imidazole;
2-[2-(5-Bromo-2-methoxy-benzylsulfanyl)-3-fluoro-phenyl]-4-methyl-4,5-dihydro-1H-imidazole;
2-[2-(5-Bromo-2-methoxy-benzylsulfanyl)-3-chloro-phenyl]-4-methyl-4,5-dihydro-1H-imidazole;
2-[2-(5-Bromo-2-methoxy-benzylsulfanyl)-3,4-difluoro-phenyl]-1,4,5,6-tetrahydro-pyrimidine;
2-[3-Fluoro-2-(naphthalen-1-ylsulfanylmethyl)-phenyl]-5,5-dimethyl-4,5-dihydro-1H-imidazole;
2-{2-[2-(5-Bromo-2-methoxy-phenyl)-1-methyl-ethyl]-phenyl}-1,4,5,6-tetrahydro-pyrimidine;
2-[2-(5-Bromo-2-methoxy benzyl sulfanyl)-3-fluoro-4-trifluoromethyl-phenyl]-4,4-dimethyl-4,5-dihydro-1H-imidazole;
2-[2-(5-Bromo-2-methoxy-benzyl sulfanyl)-3-fluoro-4-trifluoromethyl-phenyl]-5,5-dimethyl-1,4,5,6-tetrahydro-pyrimidine;
2-[3-Methoxy-2-(2-naphthalen-1-yl-ethyl)-phenyl]-1,4,5,6-tetrahydro-pyrimidine;
2-[2-(5-Bromo-2-methoxy-benzylsulfanyl)-3-chloro-phenyl]-1,4,5,6-tetrahydro-pyrimidin-5-ol;
2-{2-[2-(5-Bromo-2-methoxy-phenyl)-ethyl]-3-methoxy-phenyl}-1,4,5,6-tetrahydro-pyrimidine;
1-Amino-3-[2-(5-bromo-2-methoxy-phenyl)-7-chloro-benzo[b]thiophen-3-ylamino]-propan-2-ol;
2-[2-(1-Methyl-2-naphthalen-1-yl-ethyl)-phenyl]-1,4,5,6-tetrahydro-pyrimidine;
3-(5-Bromo-2-methoxy-benzylsulfanyl)-2-fluoro-4-(1,4,5,6-tetrahydro-pyrimidin-2-yl)-phenylamine;
2-{2-[2-(5-Bromo-2-methoxy-phenyl)-ethyl]-3-methyl-phenyl}-1,4,5,6-tetrahydro-pyrimidine;
2-[2-(5-Bromo-2-methoxy-benzylsulfanyl)-3-fluoro-phenyl]-1,4,5,6-tetrahydro-pyrimidin-5-ol;
1-Amino-3-[2-(5-bromo-2-methoxy-phenyl)-7-fluoro-benzo[b]thiophen-3-ylamino]-propan-2-ol;
2-[3-Methoxy-2-(naphthalen-1-ylsulfanylmethyl)-phenyl]-1,4,5,6-tetrahydro-pyrimidine;
2-[2-(5-Bromo-2-methoxy-benzylsulfanyl)-3-fluoro-phenyl]-1,4,5,6-tetrahydro-pyrimidin-5-ol;
2-[2-(5-Bromo-2-methoxy-benzylsulfanyl)-5-methoxy-phenyl]-1,4,5,6-tetrahydro-pyrimidine;
2-{2-[2-Chloro-6-(1,4,5,6-tetrahydro-pyrimidin-2-yl)-phenyl]-ethyl}-phenol;
2-[3-Methoxy-2-(naphthalen-1-ylsulfanylmethyl)-phenyl]-1,4,5,6-tetrahydro-pyrimidin-5-ol;
2-{2-[2-(5-Bromo-2-methoxy-phenyl)-ethyl]-3-methyl-phenyl}-5-methyl-4,5-dihydro-1H-imidazole;
2-{2-[2-(5-Bromo-2-methoxy-phenyl)-ethyl]-3-methyl-phenyl}-1,4,5,6-tetrahydro-pyrimidin-5-ol;
2-[2-(5-Bromo-2-methoxy-benzylsulfanyl)-3,4-difluoro-phenyl]-1,4,5,6-tetrahydro-pyrimidin-5-ol;
2-{2-[2-(5-Bromo-2-methoxy-phenyl)-ethyl]-3-chloro-phenyl}-4,4-dimethyl-4,5-dihydro-1H-imidazole;
2-[2-(5-Bromo-2-methoxy-benzylsulfanyl)-3-fluoro-4-methyl-phenyl]-1,4,5,6-tetrahydro-pyrimidine;
4,4-Dimethyl-2-[2-(naphthalen-1-ylmethylsulfanyl)-phenyl]-4,5-dihydro-oxazole;
2-[2-(5-Bromo-2-methoxy-benzylsulfanyl)-4-methoxy-phenyl]-1,4,5,6-tetrahydro-pyrimidine;
2-[5-(5-Bromo-2-methoxy-benzyl)-2-methyl-thiophen-3-yl]-1,4,5,6-tetrahydro-pyrimidine;
2-{2-[2-(5-Bromo-2-methoxy-phenyl)-ethyl]-thiophen-3-yl}-1,4,5,6-tetrahydro-pyrimidine;
2-{2-[2-(5-Bromo-2-methoxy-phenyl)-ethyl]-3-ethoxy-phenyl}-1,4,5,6-tetrahydro-pyrimidine;
2-{2-[2-(5-Bromo-2-methoxy-phenyl)-ethyl]-3-isopropoxy-phenyl}-1,4,5,6-tetrahydro-pyrimidine;
2-{2-[2-(5-Bromo-2-methoxy-phenyl)-ethyl]-3-fluoro-4-methoxy-phenyl}-1,4,5,6-tetrahydro-pyrimidine;
2-{2-[2-(5-Bromo-2-methoxy-phenyl)-ethyl]-3-isopropoxy-phenyl}-1,4,5,6-tetrahydro-pyrimidin-5-ol;
2-{2-[2-(5-Bromo-2-methoxy-phenyl)-ethyl]-3-methoxy-phenyl}-1,4,5,6-tetrahydro-pyrimidin-5-ol;
2-{2-[2-(5-Bromo-2-methoxy-phenyl)-ethyl]-4-methoxy-phenyl}-1,4,5,6-tetrahydro-pyrimidine;
2-{2-[2-(5-Bromo-2-methoxy-phenyl)-ethyl]-3-ethoxy-phenyl}-1,4,5,6-tetrahydro-pyrimidin-5-ol;
2-[2-(5-Bromo-2-methoxy-phenyl)-ethyl]-3-(1,4,5,6-tetrahydro-pyrimidin-2-yl)-benzonitrile;
2-{3-Benzyloxy-2-[2-(5-bromo-2-methoxy-phenyl)-ethyl]-phenyl}-1,4,5,6-betrahydro-pyrimidine;
2-{2-[2-(5-Bromo-2-methoxy-phenyl)-ethyl]-4-butyl-phenyl}-1,4,5,6-tetrahydro-pyrimidine;
2-{5-[2-(5-Bromo-2-methoxy-phenyl)-ethyl]-2,3-dihydro-benzo[1,4]dioxin-6-yl}-1,4,5,6-tetrahydro-pyrimidine;
2-{5-[2-(5-Bromo-2-methoxy-phenyl)-ethyl]-8-chloro-2,3-dihydro-benzo[1,4]dioxin-6-yl}-1,4,5,6-tetrahydro-pyrimidine;
2-{2-[2-(5-Bromo-2-methoxy-phenyl)-ethyl]-3-ethyl-phenyl}-1,4,5,6-tetrahydro-pyrimidine;
2-{2-[2-(5-Bromo-2-methoxy-phenyl)-ethyl]-3-propyl-phenyl}-1,4,5,6-tetrahydro-pyrimidine;
2-{2-[2-(5-Bromo-2-methoxy-phenyl)-ethyl]-3-butoxy-phenyl}-1,4,5,6-tetrahydro-pyrimidine;
2-{2-[2-(5-Bromo-2-methoxy-phenyl)-ethyl]-3-isobutoxy-phenyl}-1,4,5,6-tetrahydro-pyrimidine;
2-{2-[2-(5-Bromo-2-methoxy-phenyl)-ethyl]-3-butoxy-phenyl}-1,4,5,6-tetrahydro-pyrimidin-5-ol;
2-[2-(5-Bromo-2-methoxy-benzylsulfanyl)-3-fluoro-phenyl]-5-methoxy-1,4,5,6-tetrahydro-pyrimidine;
2-{2-[2-(5-Bromo-2-methoxy-phenyl)-ethyl]-3-chloro-phenyl}-1-methyl-1,4,5,6-tetrahydro-pyrimidine;
2-[2-(5-Bromo-2-methoxy-benzylsulfanyl)-3-fluoro-phenyl]-1-methyl-4,5-dihydro-1H-imidazole;
[2-(5-Bromo-2-methoxy-benzylsulfanyl)-3-fluoro-phenyl]-1-methyl-4,5-dihydro-1H-imidazole;
2-{2-[3-(1,4,5,6-Tetrahydro-pyrimidin-2-yl)-biphenyl-2-yl]-ethyl}-phenol;
2-{2-[2-(5-Bromo-2-methoxy-phenyl)-ethyl]-3-chloro-phenyl}-1-methyl-4,5-dihydro-1H-imidazole;
N-(3-Amino-propyl)-2-[2-(5-bromo-2-methoxy-phenyl)-ethyl]-6-methoxy-benzamide;
N-(3-Amino-propyl)-2-[2-(5-bromo-2-methoxy-phenyl)-ethyl]-6-methyl-benzamide;
2-[2-(5-Bromo-2-methoxy-benzylsulfanyl)-3-fluoro-phenyl]-5,6-dihydro-4H-pyrimidine-1-carboxylic acid 1-acetoxy-2-methyl-propyl ester;
2-[2-(5-Bromo-2-methoxy-benzylsulfanyl)-3-fluoro-phenyl]-5,6-dihydro-4H-pyrimidine-1-carboxylic acid 1-acetoxy-ethyl ester;
3-(5-Bromo-2-methoxy-phenyl)-5-chlolo-3,4-dihydro-isoquinolin-1-ylamine;
2-[2-[2-(5-Bromo-2-methoxy-phenyl)-ethyl]-3-(4-methoxy-benzyloxy)-phenyl]-1,4,5,6-tetrahydro-pyrimidine;
2-{2-[2-(5-Bromo-2-methoxy-phenyl)-ethyl]-3-chloro-phenyl}-5-methyl-1,4,5,6-tetrahydro-pyrimidin-5-ol;
2-[(5-Bromo-2-methoxy-phenyl)-(3-piperidin-1-yl-propylamino)-methyl]-3-chloro-6-methyl-phenol;
1-{2-[2-(5-Bromo-2-methoxy-phenyl)-ethyl]-6-methyl-benzyl}-piperazine;
1-{2-[2-(5-Bromo-2-methoxy-phenyl)-ethyl]-6-methyl-benzyl}-4-methyl-piperazine;
1-{2-[2-(5-Bromo-2-methoxy-phenyl)-ethyl]-6-methyl-benzyl}-piperidine;
{2-[2-(5-Bmmo-2-methoxy-phenyl)-ethyl]-6-methyl-benzyl}-diethyl-amine;
3-(5-Bmmo-2-methoxy-phenyl)-1,2,3,4-tetrahydro-isoquinoline;
1-{2-[2-(5-Bromo-2-methoxy-phenyl)-ethyl]-3-chloro-benzyl}-piperazine;
1-{2-[2-(5-Bromo-2-methoxy-phenyl)-ethyl]-3-chloro-benzyl}-4-methyl-piperazine;
1-{2-[2-(5-Bromo-2-methoxy-phenyl)-ethyl]-3-chloro-benzyl}-piperidine;
{2-[2-(5-Bromo-2-methoxy-phenyl)-ethyl]-3-chloro-benzyl}-diethyl-amine;
2-{2-[2-(5-Bromo-2-methoxy-phenyl)-ethyl]-3-chloro-phenyl}-4,5-dihydro-1H-imidazole;
(1-{2-[2-(5-Bromo-2-methoxy-phenyl)-ethyl]-3-chloro-benzyl}-piperidin-2-yl)-methanol;
2-{2-[2-(5-Bromo-2-methoxy-phenyl)-ethyl]-3-propoxy-phenyl}-1,4,5,6-tetrahydro-pyrimidine;
1-[2-(5-Brome-2-methoxy-benzylsulfanyl)-benzyl]-4-methyl-piperazine;
1-{2-[2-(5-Bromo-2-methoxy-phenyl)-ethyl]-6-chloro-benzyl}-piperazine;
1-{2-[2-(5-Bromo-2-methoxy-phenyl)-ethyl]-6-chloro-benzyl}-4-methyl-piperazine;
1-{2-[2-(5-Bromo-2-methoxy-phenyl)-ethyl]-6-chloro-benzyl}-piperidine;
{2-[2-(5-Bromo-2-methoxy-phenyl)-ethyl]-6-chloro-benzyl}-diethyl-amine;
1-(5-Bromo-2-methoxy-benzyl)-2-(4-methoxy-benzyl)-2,3-dihydro-1H-isoindole;
2-{2-[2-(5-Bromo-2-methoxy-phenyl)-ethyl]-3-chloro-phenyl}-5,6-dihydro-4H-pyrimidine-1-carboxylic acid 1-acetoxy-ethyl ester;
1-(2-{2-[2-(5-Bromo-2-methoxy-phenyl)-ethyl]-3-chloro-phenyl}-5,6-dihydro-4H-pyrimidin-1-yl)-ethanone;
1-{2-[2-(5-Bromo-2-methoxy-phenyl)-ethyl]-3-fluoro-benzyl}-4-methyl-piperazine;
{2-[2-(5-Bromo-2-methoxy-phenyl)-ethyl]-3-fluoro-benzyl}-diethyl-amine;
1-{2-[2-(5-Bromo-2-methoxy-phenyl)-ethyl]-3-fluoro-benzyl}-piperidine;
(1-{2-[2-(5-Bromo-2-methoxy-phenyl)-ethyl]-3-fluoro-benzyl}-piperidin-2-yl)-methanol;
4-Fluoro-N-{2-[4-(2-methoxy-phenyl)-piperazin-1-yl]-ethyl}-N-pyridin-2-yl-benzamide;
3-(5-Bromo-2-methoxy-phenyl)-2-methyl-1,2,3,4-tetrahydro-isoquinoline;
1-{2-[2-(5-Bromo-2-methoxy-phenyl)-ethyl]-3-chloro-benzyl}-piperazine;
1-{2-[2-(5-Bromo-2-methoxy-phenyl)-ethyl]-3-chloro-benzyl}-4-methyl-piperazine;
1-{2-[2-(5-Bromo-2-methoxy-phenyl)-ethyl]-3-chloro-benzyl}-piperidine;
{2-[2-(5-Bromo-2-methoxy-phenyl)-ethyl]-3-chloro-benzyl}-diethyl-amine;
2-{2-[2-(5-Bromo-2-methoxy-phenyl)-ethyl]-3-chloro-phenyl}-4,5-dihydro-1H-imidazole;
(1-{2-[2-(5-Bromo-2-methoxy-phenyl)-ethyl]-3-chloro-benzyl}-piperidin-2-yl)-methanol;
2-{2-[2-(5-Bromo-2-methoxy-phenyl)-ethyl]-3-propoxy-phenyl}-1,4,5,6-tetrahydro-pyrimidine;
1-[2-(5-Bromo-2-methoxy-benzylsulfanyl)-benzyl]-4-methyl-piperazine;
1-{2-[2-(5-Bromo-2-methoxy-phenyl)-ethyl]-chloro-benzyl}-piperazine;
1-{2-[2-(5-Bromo-2-methoxy-phenyl)-ethyl]-6-chloro-benzyl}-4-methyl-piperazine;
1-{2-[2-(5-Bromo-2-methoxy-phenyl)-ethyl]-6-chloro-benzyl}-piperidine;
{2-[2-(5-Bromo-2-methoxy-phenyl)-ethyl]-6-chloro-benzyl}-diethyl-ainine;
1-(5-Bromo-2-methoxy-benzyl)-2-(4-methoxy-benzyl)-2,3-dihydro-1H-isoindole;
2-{2-[2-(5-Bromo-2-methoxy-phenyl)-ethyl]-3-chloro-phenyl}-5,6-dihydro-4H-pyrimidine-1-carboxylic acid 1-acetoxy-ethyl ester;
1-(2-{2-[2-(5-Bromo-2-methoxy-phenyl)-ethyl]-3-chloro-phenyl}-5,6-dihydro-4H-pyrimidin-1-yl)-ethanone;
1-{2-[2-(5-Bromo-2-methoxy-phenyl)-ethyl]-3-fluoro-benzyl}-4-methyl-piperazine;
{2-[2-(5-Bromo-2-methoxy-phenyl)-ethyl]-3-fluoro-benzyl}-diethyl-amine;
1-{2-[2-(5-Bromo-2-methoxy-phenyl)-ethyl]-3-fluoro-benzyl}-piperidine;
4-Fluoro-N-{2-[4-(2-methoxy-phenyl)-piperazin-1-yl]-ethyl}-N-pyridin-2-yl-benzamide;
3-(5-Bromo-2-methoxy-phenyl)-2-methyl-1,2,3,4-tetrahydro-isoquinoline;
2-{2-[2-(5-Bromo-2-methoxy-phenyl)-ethyl]-3-chloro-phenyl}-1-ethyl-4,5-dihydro-1H-imidazole;
{2-[3-(5-Bromo-2-methoxy-phenyl)-3,4-dihydro-1H-isoquinolin-2-yl]-ethyl}-diethylamine;
1-(2-{2-[2-(5-Bromo-2-methoxy-phenyl)-ethyl]-3-chloro-phenyl}-4,5-dihydro-imidazol-1-yl)-ethanone;
2-{2-[2-(5-Bromo-2-methoxy-phenyl)-ethyl]-3-chloro-phenyl}-4,5-dihydro-imidazole-1-carboxylic acid ethyl ester;
2-{2-[2-(5-Bromo-2-methoxy-phenyl)-ethyl]-3-chloro-phenyl}-4,5-dihydro-imidazole-1-carboxylic acid isobutyl ester;
2-{2-[2-(5-Bromo-2-methoxy-phenyl)-ethyl]-3-chloro-phenyl}-4,5-dihydro-imidazole-1-carboxylic acid tert-butyl ester;
1-(2-{2-[2-(5-Bromo-2-methoxy-phenyl)-ethyl]-3-chloro-phenyl}-4,5-dihydro-imidazol-1-yl)-2,2-dimethyl-propan-1-one;
1-(5-Bromo-2-methoxy-benzyl)-2,3-dihydro-1H-isoindole;
1-(2-Methoxy-benzyl)-2-methyl-2,3-dihydro-1H-isoindole;
2-Methyl-1-naphthalen-1-ylmethyl-2,3-dihydro-1H-isoindole;
1-{2-[3-Chloro-2-(2-naphthaten-1-yl-ethyl)-phenyl]-4,5-dihydro-imidazol-1-yl}-2,2-dimethyl-propan-1-one;
{2-[1-(5-Bromo-2-methoxy-benzyl)-1,3-dihydro-isoindol-2-yl]-ethyl}-diethyl-amine;
2-[3-Chloro-2-(2-naphthalen-1-yl-ethyl)-phenyl]-1-methyl-1,4,5,6-tetrahydro-pyrimidine;
2-[3-Chloro-2-(2-naphalen-1-yl-ethyl)-phenyl]-5,6-dihydro-4H-pyrimidine-1-carboxylic acid tert-butyl ester;
2-[3-Chloro-2-(2-naphthalen-1-yl-ethyl)-phenyl]-4,5-dihydro-1H-imidazole;
1-{2-[3-Chloro-2-(2-naphthalen-1-yl-ethyl)-phenyl]-4,5-dihydro-imidazol-1-yl}-ethanone;
2-[3-Chloro-2-(2-naphthalen-1-yl-ethyl)-phenyl]-4,5-dihydro-imidazole-1-carboxylic acid isobutyl ester;
2-[3-Chloro-2-(2-naphalen-1-yl-ethyl)-phenyl]-4,5-dihydro-imidazole-1-carboxylic acid tert-butyl ester;
2-[3-Chloro-2-(2-naphthalen-1-yl-ethyl)-phenyl]-4,5-dihydro-imidazole-1-carboxylic acid ethyl ester;
2-[2-(5-Bromo-2-methoxy-phenyl)-ethyl]-N-(3-formylamino-propyl)-6-methyl-benzamide;
2-[3-Chloro-2-(2-naphthalen-1-yl-ethyl)-phenyl]-1-ethyl-4,5-dihydro-1H-imidazole;
1-(2-{2-[2-(5-Bromo-2-methoxy-phenyl)-ethyl]-3-chloro-phenyl}-5,6-dihydro-4H-pyrimidin-1-yl)-2,2-dimethyl-propan-1-one;
2-{2-[2-(5-Bromo-2-methoxy-phenyl)-ethyl]-thiophen-3-yl}-4,5-dihydro-1H-imidazole;
2-{2-[2-(5-Bromo-2-methoxy-phenyl)-ethyl]-3-chloro-phenyl}-5,6-dihydro-4H-pyrimidine-1-carboxylic acid isobutyl ester;
2-{2-[2-(5-Bromo-2-methoxy-phenyl)-ethyl]-3-chloro-phenyl}-1-isocyanomethyl-4,5-dihydro-1H-imidazole;
2-{2-[2-(5-Bromo-2-methoxy-phenyl)-ethyl]-thiophen-3-yl}-1-methyl-4,5-dihydro-1H-imidazole;
2-{2-[2-(5-Bromo-2-methoxy-phenyl)-ethyl]-thiophen-3-yl}-1-ethyl-4,5-dihydro-1H-imidazole;
3-(5-Bromo-2-methoxy-benzyl)-2-methyl-2,3-dihydro-isoindol-1-one;
4-(2-Methoxy-benzyl)-2-(4-methoxy-benzyl)-1,2,3,4-tetrahydro-isoquinoline;
4-(5-Bromo-2-methoxy-benzyl)-2-(4-methoxy-benzyl)-1,2,3,4-tetrahydro-isoquinoline;
2-{2-[2-(5-Bromo-2-methoxy-phenyl)-ethyl]-3-chloro-phenyl}-1-propyl-4,5-dihydro-1H-imidazole;
2-{2-[2-(5-Bromo-2-methoxy-phenyl)-ethyl]-3-chloro-phenyl}-3a,4,5,6,7,7a-hexahydro-1H-benzoimidazole;
5,5-Dimethyl-2-[2-(naphthalen-1-ylsulfanylmethyl)-phenyl]-4,5-dihydro-1H-imidazole;
2-[2-(5-Bromo-2-methoxy-benzylsulfanyl)-3-chloro-phenyl]-4,4-dimethyl-4,5-dihydro-1H-imidazole;
N-(5-Bromo-2-methoxy-benzyl)-N'-methyl-N-naphthalen-1-ylmethyl-ethane-1,2-diamine;
2-{2-[2-(5-Bromo-2-methoxy-phenyl)-ethyl]-3-chloro-phenyl}-1-ethyl-1,4,5,6-tetrahydro-pyrimidine;
2-[2-(5-Bromo-2-methoxy-phenyl)-ethyl]-3-chloro-N,N,N'-trimethyl-benzamidine;
2-[3-Chloro-2-(2-naphthalen-1-yl-ethyl)-phenyl]-1-methyl-4,5-dihydro-1H-imidazole;
1-Benzyl-2-{2-[2-(5-bromo-2-methoxy-phenyl)-ethyl]-3-chloro-phenyl}-4,5-dihydro-1H-imidazole;
({2-[2-(5-Bromo-2-methoxy-phenyl)-ethyl]-3-chloro-phenyl}-pyrrolidin-1-yl-methylene)-methyl-amine;
2-{2-[2-(5-Bromo-2-methoxy-phenyl)-ethyl]-3-chloro-phenyl}-1-isopropyl-4,5-dihydro-1H-imidazole;
2-{2-[2-(5-Bromo-2-methoxy-phenyl)-ethyl]-3-chloro-phenyl}-1-(2,2,2-tifluoro-ethyl)-4,5-dihydro-1H-imidazole;
2-[2-(5-Bromo-2-methoxy-benzylsulfanyl)-phenyl]-6-ethyl-1,4,5,6-tetrahydro-pyrimidine, and pharmaceutically acceptable salts thereof.

In the compounds mentioned above, the employed terms have the meaning as described below.

Alkyl is a straight chain or branched alkyl having 1, 2, 3, 4, 5 or 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 1, 2, 3, 4, 5, or 6 carbon atoms and one to three double bonds, preferably one or two double bonds, most preferably one double bound. Preferred examples of a C₂₋₆alkenyl group are ethenyl, prop-1-enyl, prop-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl.

Alkinyl is a straight chain or branched alkyl having 1, 2, 3, 4, 5, or 6 carbon atoms and one to three triple bonds, preferably one or two triple bonds, most prerably one triple bond. Preferred examples of a C₂₋₆alkinyl group are ethinyl, prop-1-inyl, prop-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl and n-hex-1,3,5-inyl.

Cycloalkyl is an alkyl ring having preferably 3, 4, 5, 6, 7 or 8 carbon atoms at the most, such as cyclopropyl, cyclobutyl, cyclopentyl, cyclohexyl, cycloheptyl or cyclooctyl, more preferably 3, 4, 5 or 6 carbon atoms.

Aryl is a monocarbocyclic moiety such as phenyl or a bicarbocyclic moiety such as naphthyl.

Heteroaryl is an aromatic moiety having 1, 2, 3, 4 or 5 carbon atoms and at least one heteratom independently selected from O, N and/or S. Heteroaryl is preferably selected from thienyl, pyrrolyl, imidazolyl, pyrazolyl, pyridyl, pyrazinyl, pyrimidinyl, pyridazinyl, isothiazolyl, isoxazyl, furanyl, and indazolyl, more preferably from thienyl, furanyl, imidazolyl, pyridyl, and pyrimidinyl.

Heterocyclyl is a saturated or unsaturated ring containing at least one heteroatom independently selected from O, N and/or S and 1, 2, 3, 4, 5, 6 or 7 carbon atoms. Preferably, heterocyclyl is a 4, 5, 6, 7 or 8-membered ring and is preferably selected from tetrahydrofuranyl, azetidinyl, pyrrolidinyl, piperidinyl, pyranyl, morpholinyl, thiomorpholinyl, more preferably from piperidinyl and pyrrolidinyl.

Halogen is a halogen atom selected from F, Cl, Br and I, preferably from F, Cl and Br.

Compounds of structural formulae (I) to (XIX) contain one or more asymmetric centers and can occur as racemates and racemic mixtures, single enantiomers, diastereomeric mixtures and individual diastereomers. The present invention is meant to comprehend all such isomeric forms of the compounds of structural formulae (I) to (XIX).

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylamino-ethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

It is further preferred, that the melanocortin-4 receptor antagonists are combined with one or more further therapeutic agent(s) selected from phenotiazines, 5HT3 receptor antagonists, dopamine antagonists, anticholinergic agents, anti histamins, histamine analogues, cannabinoids, corticosteroids, GABA receptor antagonists, NK1 receptor antagonists, alpha2 and alpha3 adrenoceptor antagonists.

The second therapeutic agent is preferably selected from the group consisting of cyclizine, dolasetron, granisetron, ondansetron, tropisetron, nabilone, scopolenine, cinnerazine, promethazine, betahistine, dexamethasone, methylprednisolone, metoclopramide, chlorpromazine, perphenazine, prochlorperazine, thiethylpiperazine, droperidole, domperidone and haloperidol.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably, melanocortin-4 receptor antagonists are administered orally or topically.

According to the present invention, the melanocortin-4 receptor antagonists are highly suitable in the treatment of nausea and emesis. It has been found that melanocortin-4 receptor antagonists combine highly wanted properties such as strong effects against cachexia induced by various disease states such as cancer, inflammation and infection. The compounds mentioned in the present invention prevent all types of emesis such as acute, delayed and anticipatory emesis. They are particular useful to prevent or reduce emesis induced by chemotherapy. Other types of emesis and nausea may be treated as well.

Due to their unique properties, melanocortin-4 receptor antagonists can be used advantageously in overcoming the problems connected to anti-emetic combination therapy with regards to differences in PK, high metabolic load, muscle wasting and potential drug interactions.

The effective dosage of active ingredient employed may vary depending on the particular compound employed, the mode of administration, the condition being treated and the severity of the condition being treated. Such dosage may be ascertained readily by a person skilled in the art.

When treating emesis and nausea induced by chemotherapy generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

### Formulation

The melanocortin-4 receptor antagonists are preferably formulated into a dosage form prior to administration.

The pharmaceutical compositions are prepared by known procedures using well-known and readily available ingredients. In making the formulations, the active ingredient is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Examples

### Assay

The preferred model to assess an anti emetic potential in animals is the cisplatin-induced emesis in the ferret in which the effects of 5HT3A and NK1 antagonists have been thoroughly investigated. The method, which detects antiemetic activity, follows that described by Gardner et al (Brit. J. Pharmacol., 116, 3158-3163, 1995).

Sixty minutes before administration of the test substance, ferrets are placed in individual stainless steel cages (40 x 50 x 34 cm) with a grid floor. Then, the animals are challenged with cisplatin (10 mg/kg i.p.), and immediately observed over a 3-hour period for:
- Number of ferrets showing retches and vomits;
- Latency to first retching (h:min:s);
- Latency to first vomiting (h:min:s);
- Retching (number of retches);
- Vomiting (number of vomits);
- Number of emesis periods;
- Mean duration of emesis periods (min:s);
- Severe behavioral side effects.
   Retching is defined as a rhythmic respiratory movement against a closed glottis, while vomiting is defined as a forced expulsion of upper gastrointestinal contents.

### Examples 1 to 4

The assay as described above was used to evaluate the anti emetic effects of melanocortin-4 receptor antagonists.

As test substance, compound A having the structure shown below was used. 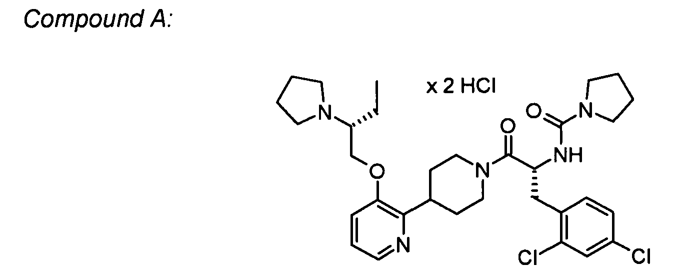 Ondansetron which is a well-known anti-emetic drug, was used for comparison reasons. Ondansetron, in line with numerous other highly selective 5HT3 antagonists, produces its anti-emetic effects through an inhibition of 5HT3 receptors (see e.g. Drug Ther. Bull. 2005, Aug;43(8):57-62). The IC₅₀ of ondansetron at the 5HT3 receptor is about 10 nanomolar. Ondansetron doesn't block the MC-4 receptor, the separation between 5HT3 and MC-4 is more than 10000 fold (internal finding).

Eight ferrets were studied per group. The test substance was evaluated at two doses (30 and 100 mg/kg), administered p.o. 90 minutes before cisplatin, and compared with an appropriate vehicle control group (vehicle administered 90 minutes before cisplatin).

Ondansetron (16 mg/kg, dissolved in distilled water, p.o.), administered p.o. 60 minutes before cisplatin, was used as reference substance. The experiment therefore included the 4 following groups:
- Group 1: test substance A (30 mg/kg, administered **90** minutes before cisplatin)
- Group 2: test substance A (100 mg/kg, administered **90** minutes before cisplatin)
- Group 3: Vehicle (10% hydroxypropyl-β-cyclodextrane in 100mM NaCl, administered **90** minutes before cisplatin)
- Group 4: Ondansetron (16 mg/kg, in distilled water, administered **60** minutes before cisplatin)

Quantitative data was analyzed by comparing treated groups with vehicle control using unpaired Student's t tests.

Comparison between treated groups and vehicle control was done using Mann-Whitney U tests.

The results are shown in Table 1 to 4.

### Results

**Table 1: The effects of vehicle on cisplatin induced emesis in the ferret**

| **Treatment (mg/kg) p.o.** | **Treatment (mg/kg) i.p. 1.5 h after p.o. administration** | **Animal number** | **Latency to first retching (h:min:s)** | **Latency to first vomiting (h:min:s)** | **Number of** | | | **Emesis mean duration (min:s)** |
|---|---|---|---|---|---|---|---|---|
| | | | | | **Retches** | **Vomits** | **Emesis periods** | |
| Vehicle | CISPLATIN 10 | 17 | - | - | 0 | 0 | 0 | - |
| | | 18 | 1:19:07 | 1:26:30 | 69 | 2 | 6 | 00:13.3 |
| | | 19 | 0:55:23 | 0:58:12 | 150 | 12 | 17 | 00:12.0 |
| | | 20 | 1:06:36 | 1:07:42 | 96 | 6 | 14 | 00:13.9 |
| | | 21 | 0:54:50 | 1:22:55 | 85 | 1 | 11 | 00:12.5 |
| | | 22 | 1:12:42 | 1:19:31 | 113 | 4 | 14 | 00:13.4 |
| | | 23 | 1:07:34 | 1:17:45 | 76 | 2 | 14 | 00:12.3 |
| | | 24 | 1:08:02 | 1:10:10 | 210 | 7 | 17 | 00:19.4 |
| Mean ± s.e.m. | | | **1:06:19** | **1:14:41** | **99.9** | **4.3** | **11.6** | **00:13:8** |
| | | | 0:03:19 | 0:03:43 | 21.8 | 1.4 | 2.1 | 0:01.0 |

The animals were observed over a period of 3 hours and the number of retches and vomits were recorded.

**Table 2: The effects of Compound A on cisplatin induced emesis in the ferret.**

| **Treatment (mg/kg) p.o.** | **Treatment (mg/kg) i.p. 1.5 h after p.o. administration** | **Animal number** | **Latency to first retching (h:min:s)** | **Latency to first vomiting (h:min:s)** | **Number of** | | | **Emesis mean duration (min:s)** |
|---|---|---|---|---|---|---|---|---|
| | | | | | **Retches** | **Vomits** | **Emesis periods** | |
| | | 1 | 1:46:16 | - | 26 | 0 | 4 | 00:11.3 |
| | | 2 | 1:29:48 | 1:30:12 | 90 | 2 | 7 | 00:19.0 |
| Compound | | | | | | | | |
| A | CISPLATIN | 3 | - | - | 0 | 0 | 0 | - |
| | | 4 | 1:22:45 | 1:23:02 | 41 | 1 | 3 | 00:19.0 |
| 30 | 10 | 5 | 0:00:13 | 0:00:24 | 118 | 2 | 13 | 00:23.1 |
| | | 6 | 1:04:49 | 1:05:05 | 84 | 5 | 10 | 00:20.3 |
| | | 7 | - | - | 0 | 0 | 0 | - |
| | | 8 | 1:32:04 | 1:36:46 | 156 | 9 | 14 | 00:15.2 |
| | | | | | | | | |
| Mean ± s.e.m. | | | **1:12:39** | **1:07:06** | **64.4** | **2.4** | **6.4** | **00:18.0** |
| | | | 0:15:30 | 0:17:30 | 20.1 | 1.1 | 2.0 | 00:01.7 |
| | | | | | | | | |
| U value (b) | | | | | 23.0 | 21.0 | 14.5 | |
| p value | | | | | 0.3431 | 0.2406 | 0.0631 | |
| | | | | | NS | NS | NS | |
| t value (b) | | | 0.431 | 0.500 | | | | 2.215 |
| p value | | | 0.6747 | 0.6278 | | | | 0.0487 |
| | | | NS | NS | | | | * |

The animals were observed over a period of 3 hours and the number of retches and vomits were recorded. The values were compared to the values produced in the vehicle control experiment (Table 1) Statistical analysis of the number of retches and vomits were done using a Mann-Whitney U-Test, the comparative analysis of the latencies to first retching and vomiting, and the duration of emesis periods was done using Student's T-test.

**Table 3: The effects of Compound A on cisplatin induced emesis in the ferret.**

| **Treatment (mg/kg) p.o.** | **Treatment (mg/kg) i.p. 1.5 h after p.o. administration** | **Animal number** | **Latency to first retching (h:min:s)** | **Latency to first vomiting (h:min:s)** | **Number of** | | | **Emesis mean duration (min:s)** |
|---|---|---|---|---|---|---|---|---|
| | | | | | **Retches** | **Vomits** | **Emesis periods** | |
| | | 9 | 1:30:40 | 1:31:17 | 28 | 2 | 5 | 00:08.0 |
| | | 10 | - | - | 0 | 0 | 0 | - |
| Compound A | CISPLATIN | 11 | - | - | 0 | 0 | 0 | - |
| | | 12 | 1:53:13 | - | 10 | 0 | 1 | 00:14.0 |
| 100 | 10 | 13 | - | - | 0 | 0 | 0 | - |
| | | 14 | - | - | 0 | 0 | 0 | - |
| | | 15 | 0:06:21 | 0:06:41 | 83 | 2 | 8 | 00:14.7 |
| | | 16 | 0:10:18 | 0:10:34 | 38 | 2 | 4 | 00:20.3 |
| Mean | | | **0:55:08** | **0:36:11** | **19.9** | **0.8** | **2.3** | **00:14.2** |
| ± s.e.m. | | | 0:27:26 | 0:27:35 | 10.4 | 0.4 | 1.1 | 00:02.5 |
| U value (b) | | | | | 8.0 | 11.5 | 7.0 | |
| p value | | | | | 0.0105 | 0.0247 | 0.0075 | |
| | | | | | * | * | ** | |
| t value (b) | | | 0.550 | 2.200 | | | | 0.188 |
| p value | | | 0.5959 | 0.0590 | | | | 0.8549 |
| | | | NS | NS | | | | NS |

**Mann-Whitney U test** (number of retches, vomits or emesis periods): * = p < 0.05; ** = p < 0.01; *** = p < 0.001

**Student's t test** (retching or vomits latency, or emesis mean duration): NS = Not Significant
(a): compared with vehicle without cisplatin.
(b): compared with vehicle with cisplatin.

The animals were observed over a period of 3 hours and the number of retches and vomits were recorded. The values were compared to the values produced in the vehicle control experiment (Table 1) Statistical analysis of the number of retches and vomits were done using a Mann-Whitney U-Test, the comparative analysis of the latencies to first retching and vomiting, and the duration of emesis periods was done using Student's T-test.

**Table 4: The effects of Ondansetron on cisplatin induced emesis in the ferret.**

| **Treatment (mg/kg) p.o.** | **Treatment (mg/kg) i.p. 1 h after p.o. administration** | **Animal number** | **Latency to first retching (h:min:s)** | **Latency to first vomiting (h:min:s)** | **Number of** | | | **Emesis mean duration (min:s)** |
|---|---|---|---|---|---|---|---|---|
| | | | | | **Retches** | **Vomits** | **Emesis periods** | |
| | | 25 | - | - | 0 | 0 | 0 | - |
| | | 26 | 1:39:22 | - | 113 | 0 | 14 | 00:14.5 |
| ONDANSETRON | CISPLATIN | 27 | 1:49:43 | 1:49:59 | 26 | 2 | 3 | 00:07.3 |
| | | 28 | - | - | 0 | 0 | 0 | - |
| 16 | 10 | 29 | - | - | 0 | 0 | 0 | - |
| | | 30 | 0:47:18 | 0:52:50 | 56 | 6 | 9 | 00:10.2 |
| | | 31 | 0:48:25 | 1:01:23 | 134 | 7 | 14 | 00:14.4 |
| | | 32 | - | - | 0 | 0 | 0 | - |
| Mean | | | **1:16:12** | **1:14:44** | **41.1** | **1.9** | **5.0** | **00:11.6** |
| ± s.e.m. | | | 0:16:30 | 0:17:48 | 19.4 | 1.0 | 2.2 | 00:01.7 |
| U value (b) | | | | | 15.5 | 17.5 | 14.0 | |
| p value | | | | | 0.0784 | 0.1162 | 0.0512 | |
| | | | | | NS | NS | NS | |
| t value (b) | | | 0.775 | 0.005 | | | | 1.226 |
| p value | | | 0.4584 | 0.9965 | | | | 0.2512 |
| | | | NS | NS | | | | NS |

**Mann-Whitney U test** (number of retches, vomits or emesis periods): NS = Not significant

**Student's t test** (retching or vomits latency, or emesis mean duration): NS = Not Significant
(a): compared with vehicle without cisplatin.
(b): compared with vehicle with cisplatin.

The animals were observed over a period of 3 hours and the number of retches and vomits were recorded. The values were compared to the values produced in the vehicle control experiment (Table 1). Statistical analysis of the number of retches and vomits were done using a Mann-Whitney U-Test, the comparative analysis of the latencies to first retching and vomiting, and the duration of emesis periods was done using Student's T-test.

The observations in the emesis model in the ferret clearly indicate that compound A has pronounced anti-emetic properties. At 30 and 100 mg/kg it dose-dependently reduced the number of retches and vomits and reduced the number of vomiting periods. The mean number of retches decreased from 99.9 (vehicle control) to 64.4 (30 mg/kg) to 19.9 (100 mg/kg). The mean number of vomits decreased from 4.3 (vehicle) to 2.4 (30 mg/kg) to 0.8 (100 mg/kg). The mean number of emesis periods decreased from 11.6 (vehicle) to 6.4 (30 mg/kg) to 2.3 (100 mg/kg). The optimal dose of 16 mg/kg of ondansetron reduced the mean number of retches from 99.9 (vehicle) to 41.1, the mean number of vomits from 4.3 to 1.9 and the number of emesis periods from 11.6 to 5.0. in other words, the strength of the anti-emetic effects of compound A at 100 mg/kg was found to be distinctly higher than the effects of ondansetron at its most active dose (Porsolt Laboratories internal standard for comparison). It has to be noted, that even though a clear anti-emetic effect was observed with ondansetron, the effect did not reach the level of statistical significance, whereas Compound A, at 100 mg/kg, showed highly significant effects on all three parameters discussed above.

## Claims

1. Melanocortin-4 receptor antagonists for the treatment of nausea and emesis.

2. Melanocortin-4 receptor antagonists according to claim 1 for the treatment of acute, delayed, post-operative, late phase and anticipatory emesis and nausea.

3. Melanocortin-4 receptor antagonists according to claim 1 for the treatment of nausea and emesis induced by chemotherapy.

4. Melanocortin-4 receptor antagonists according to claim 1 for the treatment of nausea and emesis induced by dysmenorrhoe, migraine, cancer or other pain conditions.

5. Melanocortin-4 receptor antagonists according to claim 1 for the treatment of nausea and emesis induced by radiation, toxins, pregnancy, alcohol withdrawal, drug withdrawal, nicotine withdrawal, vestibular disorders, motion sickness, post-operative sickness, surgery, gastrointestinal obstruction, reduced gastrointestinal mobility, visceral pain or increased or decreased intracranial pressure.

6. Melanocortin-4 receptor antagonists according to any of claims 1 to 5 wherein the melanocortin-4 receptor antagonists are selected from the group consisting of compounds of the general structural formulae (I) to (V): 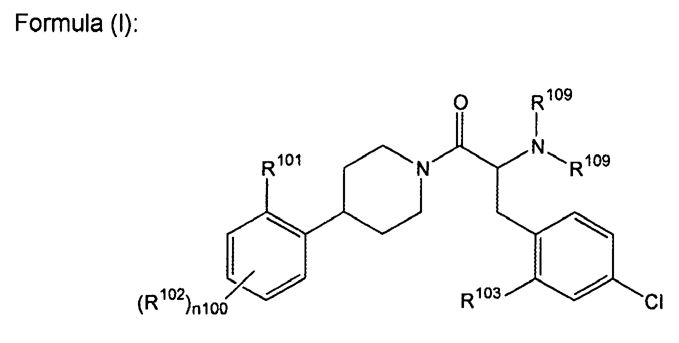 and enantiomers, diastereomers, tautomers, solvates and pharmaceutically acceptable salts thereof,
wherein
R¹⁰¹ is -(C(R¹⁰⁸)₂)_{I100}-T¹⁰⁰,
-O-(C(R¹⁰⁸)₂)ₘ₁₀₀-T¹⁰⁰,
T¹⁰⁰ is NR¹⁰⁵R¹⁰⁶,
morpholine, 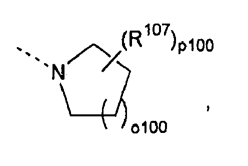 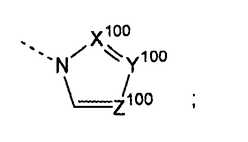
R¹⁰⁵ and R¹⁰⁶ are independently
H
C₁₋₆-alkyl,
C₂₋₆-alkenyl,
C₂₋₆-alkinyl,
C₂₋₆-alkylene-O-C₁₋₆-alkyl
wherein each alkyl, alkenyl and alkinyl is optionally substituted by one or more substituents selected from halogen atoms, CN and OH,
R¹⁰⁷ is halogen,
CN,
OH,
C₁₋₆-alkyl, optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
R¹⁰⁸ is independently
H,
F,
OH,
OMe,
C₁₋₆-alkyl, optionally substituted with 1 to 3 substituents selected from halogen, CN, OH and OMe,
C₃₋₆-cycloalkyl, optionally substituted with 1 to 3 substituents selected from halogen, CN, OH and OMe,
R¹⁰⁹ is independently
H,
C₁₋₆-alkyl, optionally substituted with 1 to 3 substituents selected from halogen, CN, OH and OMe,
C₃₋₆-cycloalkyl, optionally substituted with 1 to 3 substituents selected from halogen, CN, OH and OMe,
X¹⁰⁰ is CH, N,
Y¹⁰⁰ is CH, N,
Z¹⁰⁰ is CH, N,
R¹⁰² is F,
Cl,
methyl,
R¹⁰³ is Cl,
methyl,
I100 is 3, 4,
m100 is 2, 3, 4,
n100 is 0, 1, 2, 3, 4,
o100 is 0, 1, 2,
p100 is 0, 1, 2, 3, 4; 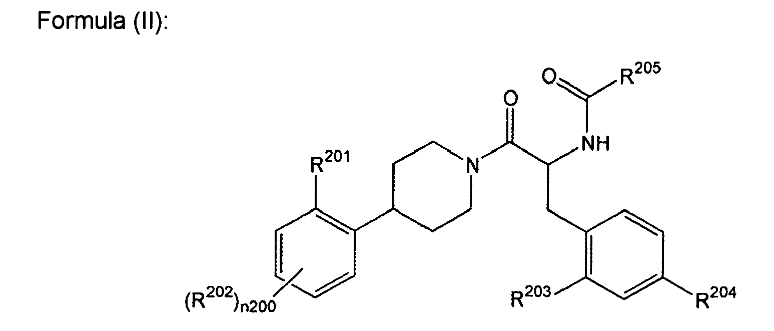 and the enantiomers, diastereomers, tautomers, solvates and pharmaceutically acceptable salts thereof,
wherein
R²⁰¹ is -(C(R²⁰⁶)₂)_{I200}-T²⁰⁰, or
-O-(C(R²⁰⁶)₂)ₘ₂₀₀-T²⁰⁰;
R²⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cydoalkyl, optionally substituted with 1 to 3 substituents selected from halogen, CN, OH and OCH₃;
T²⁰⁰ is NR²⁰⁷R²⁰⁸,
morpholine, 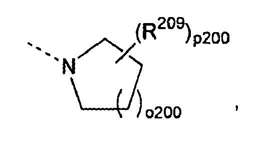 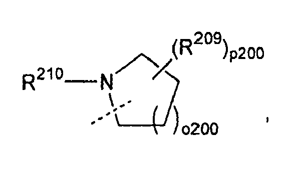 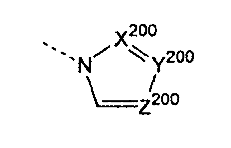 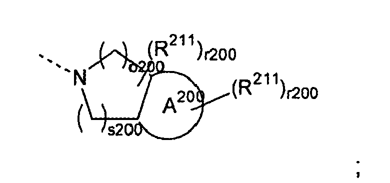
R²⁰⁷ and R²⁰⁸ are independently from each other selected from
H,
C₁₋₆-alkyl,
C₂₋₆-alkenyl
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R²⁰⁹ is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
R²¹⁰ is H, or
C₁-C₆-alkyl;
R²¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X²⁰⁰ is CH or N;
Y²⁰⁰ is CH or N;
Z²⁰⁰ is CH or N;
A²⁰⁰ is a 3-7-membered saturated, unsaturated or aromatic ring containing 0-2 nitrogen atoms;
R²⁰² is independently selected from
F,
Cl,
CH₃, and
CF₃;
R²⁰³ is H,
Cl,
F, or
CH₃;
R²⁰⁴ is Cl or F;
R²⁰⁵ is 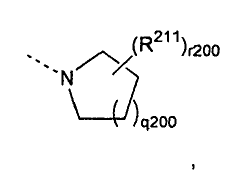 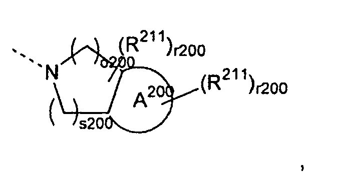 morpholine, optionally substituted by 1 to 3, same or different substituents
R²¹⁴ or
NR²¹²R²¹³;
R²¹² and R²¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R²¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂,
I200 is 1, 2, 3, or 4;
m200 is 0, 1, 2, 3, or 4;
n200 is 0, 1, 2, 3, or 4;
o200 is 0, 1, or 2;
p200 is 0, 1, 2, 3, or 4;
q200 is 0, 1, 2, or 3;
r200 is 0, 1, 2, 3, or 4 and
s200 is 1, or 2; 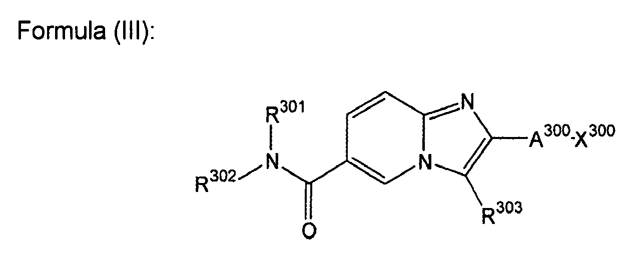 and enantiomers, diastereomers, tautomers, solvates and pharmaceutically acceptable salts thereof,
wherein
R³⁰¹ and R³⁰² are independently from each other selected from
H,
C₁₋₆ alkyl,
C₁₋₆ alkylene-O-C₁₋₆alkyl,
C₁₋₃ alkylene-heterocyclyl, and
C₁₋₆ alkylene-C₃₋₇cycloalkyl, or
R³⁰¹ and R³⁰² form together with the nitrogen atom to which they are attached a 5 to 6-membered ring which may additionally contain one oxygen atom in the ring and which ring is unsubstituted or substituted by one or more substituents selected from OH, C₁₋₆alkyl, O-C₁₋₆alkyl, C₀₋₃alkylene-C₃₋₅cycloalkyl, C₁₋₆alkylene-O-C₁₋₆alkyl or (CH₂)₀₋₃-phenyl;
A³⁰⁰ is -NH-,
-C₁₋₆alkylene,
-C₂₋₆alkenylene,
-C₂₋₆alkinylene or
a bond,
wherein alkylene, alkenylene and alkinylene are unsubstituted or substituted with one ore more R³⁰⁷;
R³⁰⁷ is independently selected from
H,
C₁₋₆alkyl,
OR³¹⁴,
NR^{315a}R^{315b},
halogen,
phenyl and
heteroaryl,
wherein phenyl and heteroaryl are unsubstituted or substituted by 1 to 3
R^{304a};
X³⁰⁰ is H,
CN,
phenyl,
phenyl which is fused with a saturated heterocyclic 6-membered ring, wherein the heterocyclic ring may contain 1 or 2 heteroatoms selected from O and N and wherein the heterocyclic ring may further be optionally substituted by an oxo group,
4 to 8-membered saturated or unsaturated heterocyclyl containing 1, 2, 3 or 4 heteroatoms independently selected from N, O and S,
5- to 6-membered heteroaryl containing 1, 2, 3, or 4 heteroatoms independently selected from N, O and S, or
-C(O)-R³⁰⁶,
C₃₋₈cycloalkyl, unsubstituted or substituted with one or more halogen atoms,
-OR³¹⁴,
halogen or
NR^{315a}R^{315b},
wherein each phenyl, heterocyclyl and heteroaryl is optionally substituted by 1 to 3 R^{304a} and/or 1 R^{304b} and/or 1 R³⁰⁵;
R^{304a} is halogen,
CN,
C₁₋₆alkyl, optionally substituted with one or more halogen atoms,
O-C₁₋₆alkyl, optionally substituted with one or more halogen atoms, or
OH;
R^{304b} is C(O)NH₂,
C(O)NH-C₁₋₆alkyl,
C(O)N-(C₁₋₆alkyl)₂,
SO₂-C₁₋₆alkyl,
C(O)NH-SO₂-C₁₋₆alkyl,
oxo, whereby the ring is at least partially saturated,
NH₂,
NH-C₁₋₆alkyl,
N-(C₁₋₆alkyl)₂,
NH-SO₂-CH₃, or
NH-SO₂-CF₃;
R³⁰⁵ is 5 to 6-membered saturated or unsaturated heterocyclyl containing 1 to 3 heteroatoms independently selected from N, O and S or
5 to 6-membered heteroaryl containing 1 to 3 heteroatoms independently selected from N, O and S,
wherein the heterocyclyl and the heteroaryl are optionally substituted by 1 or 2 R^{304a};
R³⁰⁶ is H,
C₁₋₆alkyl, optionally substituted with one or more halogen atoms, phenyl or
4 to 8-membered saturated or unsaturated heterocyclyl containing 1 to 3 heteroatoms selected from N, O and S,
wherein each phenyl and heterocyclyl is optionally substituted by 1 to 3 R^{304a} and/or 1 R³⁰⁵,
OH,
O-C₁₋₆alkyl, wherein alkyl is unsubstituted or substituted with one or more
R³¹⁶ or
NR^{316a}R^{316b};
R³⁰³ is -(CR³⁰⁸R³⁰⁹)ₙ₃₀₀-T³⁰⁰;
R³⁰⁸ and R³⁰⁹ are independently from each other selected from
H,
OH,
halogen,
C₁₋₆alkyl, and
O-C₁₋₆ alkyl;
n300 is 1 to 6;
T³⁰⁰ is 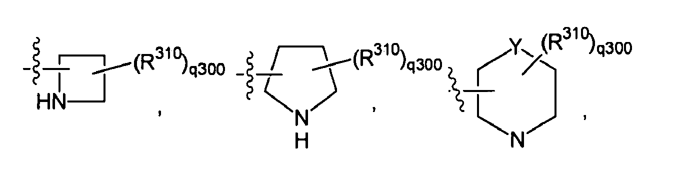 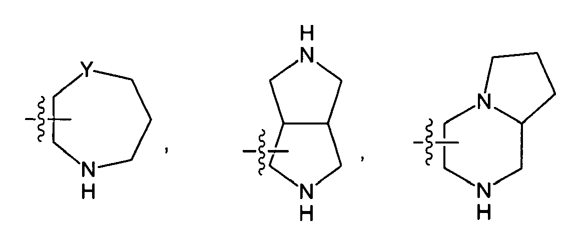 or NR³¹²R³¹³;
R³¹⁰ is H,
NH₂,
OH,
C₁₋₆alkyl,
halogen,
NH(C₁₋₆alkyl),
N(C₁₋₆alkyl)₂,
phenyl or
heteroaryl,
wherein phenyl and heteroaryl are optionally substituted by 1 to 3 R^{304a};
q300 is 1 or 2
Y³⁰⁰ is CH₂, NR³¹¹ or O;
R³¹¹ is H,
C₁₋₆alkyl or
(CH₂)₀₋₆-C₃₋₇cycloalkyl;
R³¹² and R³¹³ are independently from each other selected from
H,
C₁₋₆ alkyl,
(CH₂)₀₋₂-C₃₋₇cycloalkyl and
C₁₋₆alkylene-O-C₁₋₆alkyl;
wherein C₁₋₆alkyl, C₁₋₆alkylene and C₃₋₇cycloalkyl are optionally substituted by 1 to 3 R^{304a};
R³¹⁴ is H or
C₁₋₆alkyl, unsubstituted or substituted with one or more substituents selected from halogen,
phenyl or heteroaryl,
wherein phenyl and heteroaryl are optionally substituted by 1 to 3 R^{304a};
R^{315a} and R^{315b} are independently from each other selected from
H,
C₁₋₆alkyl, unsubstituted or substituted with one or more substituents selected from halogen, OH, O(C₁₋₆alkyl), NH₂, NH(C₁₋₆alkyl) and N(C₁₋₆ alkyl)₂,
phenyl or heteroaryl,
wherein phenyl and heteroaryl are optionally substituted by 1 to 3 R^{304a} and
C(O)C₁₋₆alkyl;
R³¹⁶, R^{316a} and R^{316b} are independently from each other selected from
H,
C₁₋₆alkyl, unsubstituted or substituted with halogen, OH, O(C₁₋₆alkyl),
NH₂, NH(C₁₋₆alkyl), N(C₁₋₆ alkyl)₂,
C₀₋₃alkylene-C₃₋₅cycloalkyl,
phenyl and
heteroaryl,
wherein phenyl and heteroaryl are unsubstituted or substituted by 1 to 3
R^{304a}; 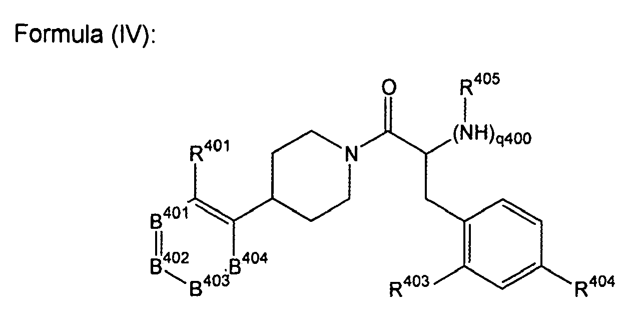 and the enantiomers, diastereomers, tautomers, solvates and pharmaceutically acceptable salts thereof,
wherein
R⁴⁰¹ is -N(R⁴¹⁰)-(C(R⁴⁰⁶)₂)ₘ₄₀₀-T⁴⁰⁰
-(C(R⁴⁰⁶)₂)_{I400}-T⁴⁰⁰, or
-O-(C(R⁴⁰⁶)₂)ₘ₄₀₀-T⁴⁰⁰;
R⁴⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T⁴⁰⁰ is NR⁴⁰⁷R⁴⁰⁸,
morpholine, 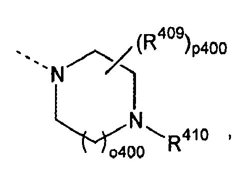 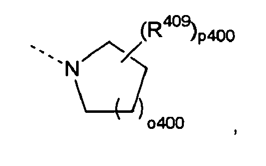 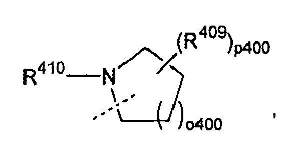 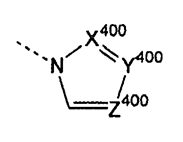 or 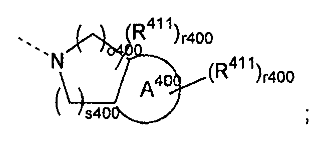
R⁴⁰⁷ and R⁴⁰⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁴⁰⁹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH, and
C₁₋₆-alkylene-O-C₁₋₆-alkyl optionally substituted with 1 to 3 substituents selected from halogen, CN and OH;
R⁴¹⁰ is H, or
C₁-C₆-alkyl;
R⁴¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X⁴⁰⁰ is CH or N;
Y⁴⁰⁰ is CH or N;
Z⁴⁰⁰ is CH or N;
A⁴⁰⁰ is a 3-7-membered saturated, unsaturated or aromatic ring containing 0-2 nitrogen atoms;
B⁴⁰¹ to B⁴⁰⁴ are independently selected from CR⁴⁰² and N;
R⁴⁰² is independently selected from
H,
F,
Cl,
CH₃,
OCH₃, and
CF₃;
R⁴⁰³ is H,
Cl,
F, or
CH₃;
R⁴⁰⁴ is Cl or F;
R⁴⁰⁵ is 4 to 7-membered heterocyclyl containing 1 or 2 heteroatoms independently selected from the group consisting of NR⁴¹², O and S; or
4 to 7-membered heteroaryl containing 1 to 4 heteroatoms independently selected from the group consisting of N, O and S;
wherein each heterocyclyl may be unsubstituted or substituted by 1 or 2 substituents selected from the group consisting of oxo group, CN, C₁₋₆-alkyl, C₃₋₆-cycloalkyl, halogen and OH, and
each heteroaryl may be unsubstituted or substituted by 1 or 2 substituents selected from the group consisting of C₁₋₆-alkyl, C₃₋₆-cycloalkyl, O-C₁₋₆-alkyl, halogen and CN;
R⁴¹² is hydrogen,
C₁₋₆-alkyl,
C₃₋₆-cycloalkyl and
C(O)C₁₋₆-alkyl;
l400 is 0, 1, 2, 3, or 4;
m400 is 0, 1, 2, 3, or 4;
o400 is 0, 1, or 2;
p400 is 0, 1, 2, 3, or 4;
q400 is 0, or 1;
r400 is 0, 1, 2, 3, or 4 and
s400 is 1, or 2; 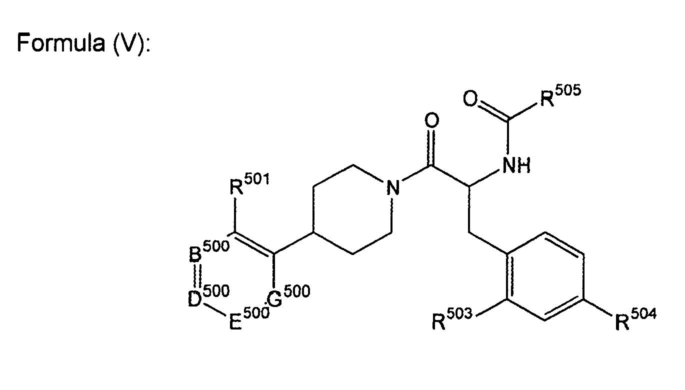 and the enantiomers, diastereomers, tautomers, solvates and pharmaceutically acceptable salts thereof,
wherein
R⁵⁰¹ is -N(R⁵¹⁰)-(C(R⁵⁰⁶)₂)ₘ₅₀₀-T⁵⁰⁰
-(C(R⁵⁰⁶)₂)_{I500}-T⁵⁰⁰, or
-O-(C(R⁵⁰⁶)₂)ₘ₅₀₀-T⁵⁰⁰;
R⁵⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T⁵⁰⁰ is NR⁵⁰⁷R⁵⁰⁸,
morpholine, 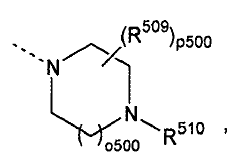 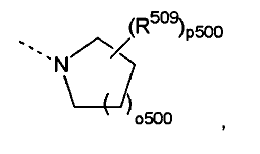 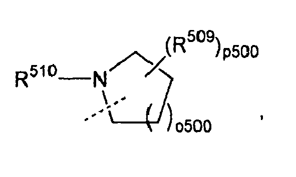 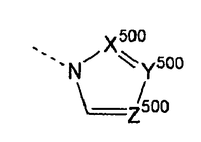 or 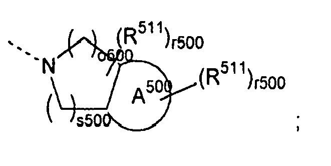
R⁵⁰⁷ and R⁵⁰⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or
more halogen atoms, CN or OH;
R⁵⁰⁹ is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents
selected from halogen, CN and OH;
R⁵¹⁰ is H, or
C₁-C₆-alkyl;
R⁵¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X⁵⁰⁰ is CH or N;
Y⁵⁰⁰ is CH or N;
Z⁵⁰⁰ is CH or N;
A⁵⁰⁰ is a 3-7-membered saturated, unsaturated or aromatic ring containing 0-2 nitrogen atoms;
B⁵⁰⁰ is CR⁵⁰² or N;
G⁵⁰⁰ is CR⁵⁰² or N;
D⁵⁰⁰ is CR⁵⁰² or N;
E⁵⁰⁰ is CR⁵⁰² or N;
with the proviso that one or two of the variables B⁵⁰⁰, G⁵⁰⁰, D⁵⁰⁰ and E⁵⁰⁰ must be N;
R⁵⁰² is independently selected from
H,
F,
Cl,
CH₃,
OCH₃, and
CF₃;
R⁵⁰³ is H,
Cl,
F, or
CH₃;
R⁵⁰⁴ is Cl or F;
R⁵⁰⁵ is 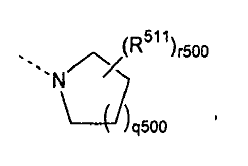 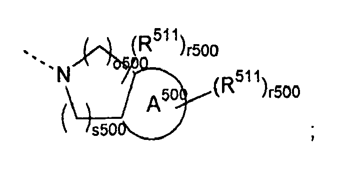 morpholine, optionally substituted by 1 to 3, same or different substituents R⁵¹⁴, or
NR⁵¹²R⁵¹³;
R⁵¹² and R⁵¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R⁵¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
I500 is 0, 1, 2, 3, or 4;
m500 is 0, 1, 2, 3, or 4;
o500 is 0, 1, or 2;
p500 is 0, 1, 2, 3, or 4;
q500 is 0, 1, 2, or 3;
r500 is 0, 1, 2, 3, or 4 and
s500 is 1, or 2.

7. Melanocortin-4 receptor antagonists for the treatment of nausea and emesis according to any of claims 1 to 6 wherein the melanocortin-4 receptor antagonists is combined with one or more further therapeutic agent(s) selected from phenotiazines, 5HT3 receptor antagonists, dopamine antagonists, anticholinergic agents, anti histamins, histamine analogues, cannabinoids, corticosteroids, GABA receptor antagonists, NK1 receptor antagonists, alpha2 and alpha3 adrenoceptor antagonists.

8. Use of melanocortin-4 receptor antagonists for the preparation of a medicament for the treatment of nausea and emesis.

9. The use according to claim 8 wherein the nausea and emesis is selected from acute, delayed, post-operative, late phase and anticipatory emesis and nausea.

10. The use according to claim 8 wherein nausea and emesis are induced by chemotherapy.

11. The use according to claim 8 wherein nausea and emesis are induced by dysmenorrhoe, migraine, cancer or other pain condition.

12. The use according to claim 8 wherein nausea and emesis are induced by radiation, toxins, pregnancy, alcohol withdrawal, drug withdrawal, nicotine withdrawal, vestibular disorders, motion sickness, post-operative sickness, surgery, gastrointestinal obstruction, reduced gastrointestinal mobility, visceral pain or increased or decreased intracranial pressure.

13. The use according to any of claims 8 to 12 wherein the melanocortin-4 receptor antagonists are selected from the group consisting of Formulae (I) to (V): 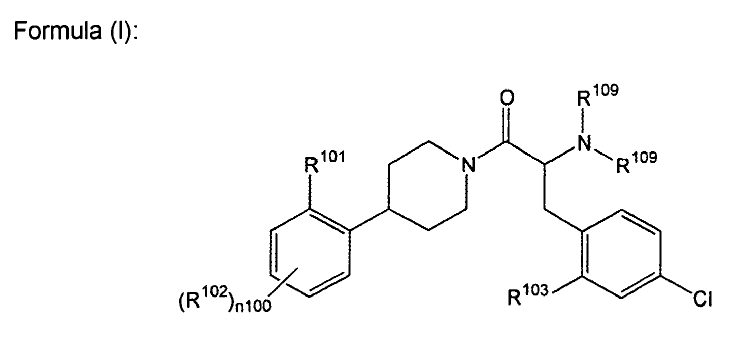 and enantiomers, diastereomers, tautomers, solvates and pharmaceutically acceptable salts thereof,
wherein
R¹⁰¹ is -(C(R¹⁰⁸)₂)_{I100}-T¹⁰⁰,
-O-(C(R¹⁰⁸)₂)ₘ₁₀₀-T¹⁰⁰,
T¹⁰⁰ is NR¹⁰⁵R¹⁰⁶,
morpholine, 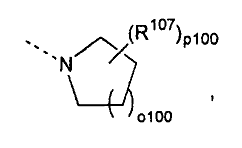 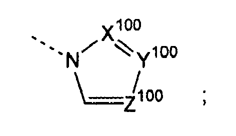
R¹⁰⁵ and R¹⁰⁶ are independently
H
C₁₋₆-alkyl,
C₂₋₆-alkenyl,
C₂₋₆-alkinyl,
C₂₋₆-alkylene-O-C₁₋₆-alkyl
wherein each alkyl, alkenyl and alkinyl is optionally substituted by one or more substituents selected from halogen atoms, CN and OH,
R¹⁰⁷ is halogen,
CN,
OH,
C₁₋₆-alkyl, optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
R¹⁰⁸ is independently
H,
F,
OH,
OMe,
C₁₋₆-alkyl, optionally substituted with 1 to 3 substituents selected from halogen, CN, OH and OMe,
C₃₋₆-cycloalkyl, optionally substituted with 1 to 3 substituents selected from halogen, CN, OH and OMe,
R¹⁰⁹ is independently
H,
C₁₋₆-alkyl, optionally substituted with 1 to 3 substituents selected from halogen, CN, OH and OMe,
C₃₋₆-cycloalkyl, optionally substituted with 1 to 3 substituents selected from halogen, CN, OH and OMe,
X¹⁰⁰ is CH, N,
Y¹⁰⁰ is CH, N,
Z¹⁰⁰ is CH, N,
R¹⁰² is F,
Cl,
methyl,
R¹⁰³ is Cl,
methyl,
I100 is 3,4,
m100 is 2, 3, 4,
n100 is 0, 1, 2, 3, 4,
o100 is 0, 1, 2,
p100is 0, 1, 2, 3, 4; 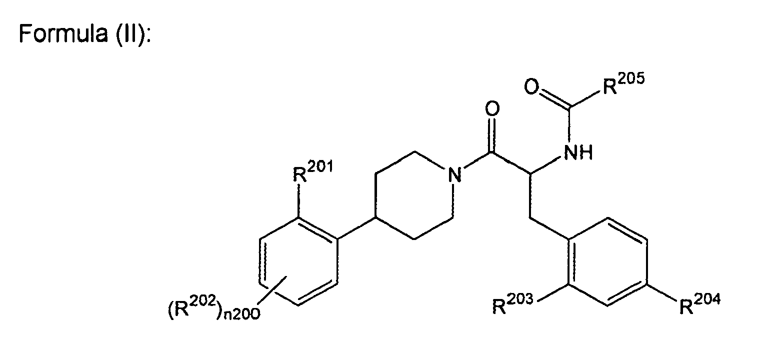 and the enantiomers, diastereomers, tautomers, solvates and pharmaceutically acceptable salts thereof,
wherein
R²⁰¹ is -(C(R²⁰⁶)₂)ₗ₂₀₀-T²⁰⁰, or
-O-(C(R²⁰⁶)₂)ₘ₂₀₀-T²⁰⁰;
R²⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T²⁰⁰ is NR²⁰⁷R²⁰⁸,
morpholine, 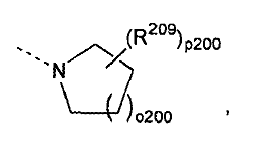 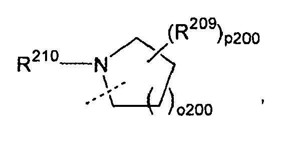 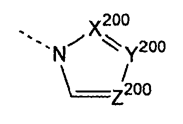 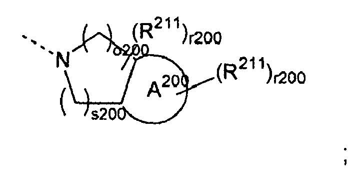
R²⁰⁷ and R²⁰⁸ are independently from each other selected from
H,
C₁₋₆-alkyl,
C₂₋₆-alkenyl
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R²⁰⁹ is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
R²¹⁰ is H, or
C₁-C₆-alkyl;
R²¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X²⁰⁰ is CH or N;
Y²⁰⁰ is CH or N;
Z²⁰⁰ is CH or N;
A²⁰⁰ is a 3-7-membered saturated, unsaturated or aromatic ring containing 0-2 nitrogen atoms;
R²⁰² is independently selected from
F,
Cl,
CH₃, and
CF₃;
R²⁰³ is H,
Cl,
F, or
CH₃;
R²⁰⁴ is Cl or F;
R²⁰⁵ is 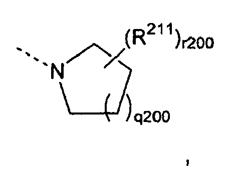 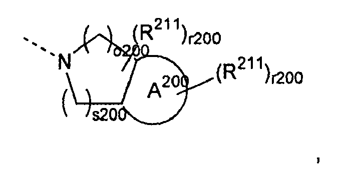 morpholine, optionally substituted by 1 to 3, same or different substituents R²¹⁴, or
NR²¹²R²¹³;
R²¹² and R²¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R²¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or
C₁₋₆-alkylene-N(C₁₋₆-alkyl)₂;
I200 is 1, 2, 3, or 4;
m200 is 0, 1, 2, 3, or 4;
n200 is 0, 1, 2, 3, or 4;
o200 is 0, 1, or 2;
p200 is 0, 1, 2, 3, or 4;
q200 is 0, 1, 2, or 3;
r200 is 0, 1, 2, 3, or 4 and
s200 is 1, or 2; 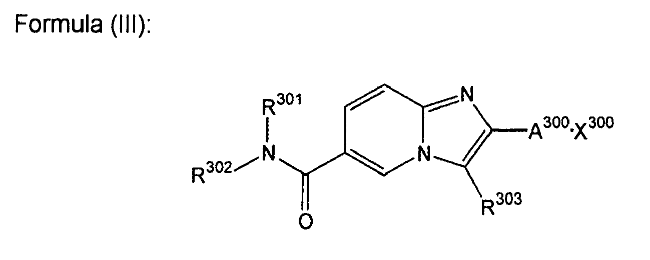 and enantiomers, diastereomers, tautomers, solvates and pharmaceutically acceptable salts thereof,
wherein
R³⁰¹ and R³⁰² are independently from each other selected from
H,
C₁₋₆ alkyl,
C₁₋₆ alkylene-O-C₁₋₆alkyl,
C₁₋₃ alkylene-heterocyclyl, and
C₁₋₆ alkylene-C₃₋₇cycloalkyl, or
R³⁰¹ and R³⁰² form together with the nitrogen atom to which they are attached a 5 to 6-membered ring which may additionally contain one oxygen atom in the ring and which ring is unsubstituted or substituted by one or more substituents selected from OH, C₁₋₆alkyl, O-C₁₋₆alkyl, C₀₋₃alkylene-C₃₋₅cycloalkyl, C₁₋₆alkylene-O-C₁₋₆alkyl or (CH₂)₀₋₃-phenyl;
A³⁰⁰ is -NH-,
-C₁₋₆alkylene,
-C₂₋₆alkenylene,
-C₂₋₆alkinylene or
a bond,
wherein alkylene, alkenylene and alkinylene are unsubstituted or substituted with one ore more R³⁰⁷;
R³⁰⁷ is independently selected from
H,
C₁₋₆alkyl,
OR³¹⁴,
NR^{315a}R^{315b},
halogen,
phenyl and
heteroaryl,
wherein phenyl and heteroaryl are unsubstituted or substituted by 1 to 3
R^{304a};
X³⁰⁰ is H,
CN,
phenyl,
phenyl which is fused with a saturated heterocyclic 6-membered ring, wherein the heterocyclic ring may contain 1 or 2 heteroatoms selected from O and N and wherein the heterocyclic ring may further be optionally substituted by an oxo group,
4 to 8-membered saturated or unsaturated heterocyclyl containing 1, 2, 3 or 4 heteroatoms independently selected from N, O and S,
5- to 6-membered heteroaryl containing 1, 2, 3, or 4 heteroatoms independently selected from N, O and S, or
-C(O)-R³⁰⁶,
C₃₋₈cycloalkyl, unsubstituted or substituted with one or more halogen atoms,
-OR³¹⁴,
halogen or
NR^{315a}R^{315b},
wherein each phenyl, heterocyclyl and heteroaryl is optionally substituted by 1 to 3 R^{304a} and/or 1 R^{304b} and/or 1 R³⁰⁵;
R^{304a} is halogen,
CN,
C₁₋₆alkyl, optionally substituted with one or more halogen atoms,
O-C₁₋₆alkyl, optionally substituted with one or more halogen atoms, or OH;
R^{304b} is C(O)NH₂,
C(O)NH-C₁₋₆alkyl,
C(O)N-(C₁₋₆alkyl)₂,
SO₂-C₁₋₆alkyl,
C(O)NH-SO₂-C₁₋₆alkyl,
oxo, whereby the ring is at least partially saturated,
NH₂,
NH-C₁₋₆alkyl,
N-(C₁₋₆alkyl)₂,
NH-SO₂-CH₃, or
NH-SO₂-CF₃;
R³⁰⁵ is 5 to 6-membered saturated or unsaturated heterocyclyl containing 1 to 3 heteroatoms independently selected from N, O and S or
5 to 6-membered heteroaryl containing 1 to 3 heteroatoms independently selected from N, O and S,
wherein the heterocyclyl and the heteroaryl are optionally substituted by 1 or 2 R^{304a};
R³⁰⁶ is H,
C₁₋₆alkyl, optionally substituted with one or more halogen atoms, phenyl or
4 to 8-membered saturated or unsaturated heterocyclyl containing 1 to 3 heteroatoms selected from N, O and S,
wherein each phenyl and heterocyclyl is optionally substituted by 1 to 3 R^{304a} and/or 1 R³⁰⁵,
OH,
O-C₁₋₆alkyl, wherein alkyl is unsubstituted or substituted with one or more R³¹⁶ or
NR^{316a}R^{316b};
R³⁰³ is -(CR³⁰⁸R³⁰⁹)ₙ₃₀₀-T³⁰⁰;
R³⁰⁸ and R³⁰⁹ are independently from each other selected from
H,
OH,
halogen,
C₁₋₆alkyl, and
O-C₁₋₆ alkyl;
n300 is 1 to 6;
T³⁰⁰ is 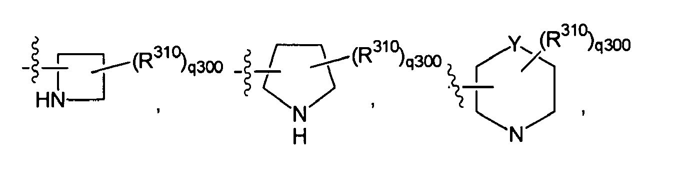 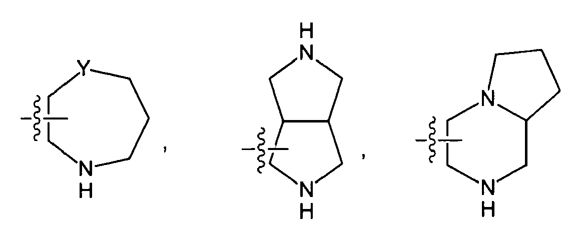 or NR³¹²R³¹³;
R³¹⁰ is H,
NH₂,
OH,
C₁₋₆alkyl,
halogen,
NH(C₁₋₆alkyl),
N(C₁₋₆alkyl)₂,
phenyl or
heteroaryl,
wherein phenyl and heteroaryl are optionally substituted by 1 to 3 R^{304a};
q300 is 1 or 2;
Y³⁰⁰ is CH₂, NR³¹¹ or O;
R³¹¹ is H,
C₁₋₆alkyl or
(CH₂)₀₋₆-C₃₋₇cycloalkyl;
R³¹² and R³¹³ are independently from each other selected from
H,
C₁₋₆ alkyl,
(CH₂)₀₋₂-C₃₋₇cycloalkyl and
C₁₋₆alkylene-O-C₁₋₆alkyl;
wherein C₁₋₆alkyl, C₁₋₆alkylene and C₃₋₇cycloalkyl are optionally
substituted by 1 to 3 R^{304a};
R³¹⁴ is H or
C₁₋₆alkyl, unsubstituted or substituted with one or more substituents selected from halogen, phenyl or heteroaryl,
wherein phenyl and heteroaryl are optionally substituted by 1 to 3 R^{304a};
R^{315a} and R^{315b} are independently from each other selected from
H,
C₁₋₆alkyl, unsubstituted or substituted with one or more substituents selected from halogen, OH, O(C₁₋₆alkyl), NH₂, NH(C₁₋₆alkyl) and N(C₁₋₆ alkyl)₂,
phenyl or heteroaryl,
wherein phenyl and heteroaryl are optionally substituted by 1 to 3 R^{304a} and
C(O)C₁₋₆alkyl;
R³¹⁶, R^{316a} and R^{316b} are independently from each other selected from
H,
C₁₋₆alkyl, unsubstituted or substituted with halogen, OH, O(C₁₋₆alkyl),
NH₂, NH(C₁₋₆alkyl), N(C₁₋₆ alkyl)₂,
C₀₋₃alkylene-C₃₋₅cycloalkyl,
phenyl and
heteroaryl,
wherein phenyl and heteroaryl are unsubstituted or substituted by 1 to 3
R^{304a}; 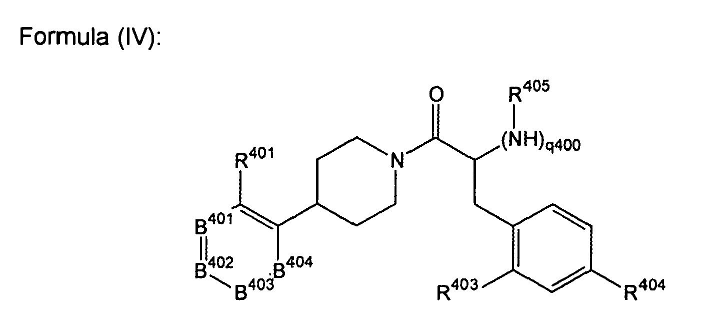 and the enantiomers, diastereomers, tautomers, solvates and pharmaceutically acceptable salts thereof,
wherein
R⁴⁰¹ is -N(R⁴¹⁰)-(C(R⁴⁰⁶)₂)ₘ₄₀₀-T⁴⁰⁰
-(C(R⁴⁰⁶)₂)ₗ₄₀₀-T⁴⁰⁰, or
-O-(C(R⁴⁰⁶)₂)ₘ₄₀₀-T⁴⁰⁰;
R⁴⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T⁴⁰⁰ is NR⁴⁰⁷R⁴⁰⁸,
morpholine, 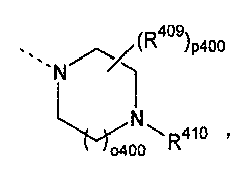 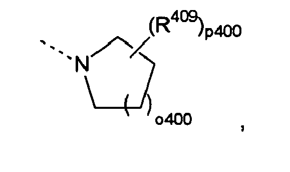 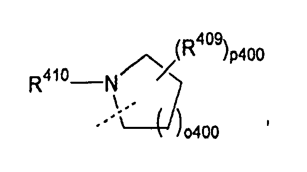 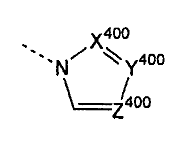 or 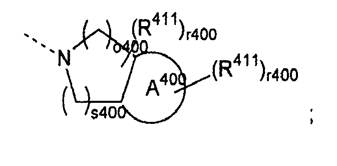
R⁴⁰⁷ and R⁴⁰⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or more halogen atoms, CN or OH;
R⁴⁰⁹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH, and
C₁₋₆-alkylene-O-C₁₋₆-alkyl optionally substituted with 1 to 3 substituents selected from halogen, CN and OH;
R⁴¹⁰ is H, or
C₁-C₆-alkyl;
R⁴¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X⁴⁰⁰ is CH or N;
Y⁴⁰⁰ is CH or N;
Z⁴⁰⁰ is CH or N;
A⁴⁰⁰ is a 3-7-membered saturated, unsaturated or aromatic ring containing 0-2 nitrogen atoms;
B⁴⁰¹ to B⁴⁰⁴ are independently selected from CR⁴⁰² and N;
R⁴⁰² is independently selected from
H,
F,
Cl,
CH₃,
OCH₃, and
CF₃;
R⁴⁰³ is H,
Cl,
F, or
CH₃;
R⁴⁰⁴ is Cl or F;
R⁴⁰⁵ is 4 to 7-membered heterocyclyl containing 1 or 2 heteroatoms independently selected from the group consisting of NR⁴¹², O and S; or
4 to 7-membered heteroaryl containing 1 to 4 heteroatoms independently selected from the group consisting of N, O and S;
wherein each heterocyclyl may be unsubstituted or substituted by 1 or 2 substituents selected from the group consisting of oxo group, CN, C₁₋₆-alkyl, C₃₋₆-cycloalkyl, halogen and OH, and
each heteroaryl may be unsubstituted or substituted by 1 or 2 substituents selected from the group consisting of C₁₋₆-alkyl, C₃₋₆-cycloalkyl, O-C₁₋₆-alkyl, halogen and CN;
R⁴¹² is hydrogen,
C₁₋₆-alkyl,
C₃₋₆-cycloalkyl and
C(O)C₁₋₆-alkyl;
l400 is 0, 1, 2, 3, or 4;
m400 is 0, 1, 2, 3, or 4;
o400 is 0, 1, or 2;
p400 is 0, 1, 2, 3, or 4;
q400 is 0, or 1;
r400 is 0, 1, 2, 3, or 4 and
s400 is 1, or 2; 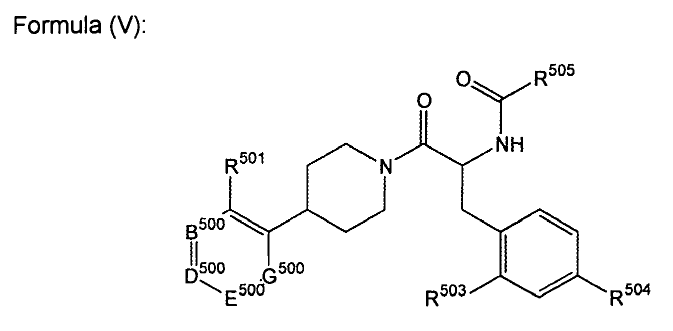 and the enantiomers, diastereomers, tautomers, solvates and pharmaceutically acceptable salts thereof,
wherein
R⁵⁰¹ is -N(R⁵⁰¹)-(C(R⁵⁰⁶)₂)ₘ₅₀₀-T⁵⁰⁰
-(C(R⁵⁰⁶)₂)ₗ₅₀₀-T⁵⁰⁰, or
-O-(C(R⁵⁰⁶)₂)ₘ₅₀₀-T⁵⁰⁰;
R⁵⁰⁶ is independently selected from
H,
F,
OH,
OCH₃,
C₁₋₆-alkyl, optionally substituted with 1 to 3 substituents selected from halogen, CN, OH and OCH₃, and
C₃₋₆-cycloalkyl, optionally substituted with 1 to 3 substituents selected from halogen, CN, OH and OCH₃;
T⁵⁰⁰ is NR⁵⁰⁷R⁵⁰⁸,
morpholine, 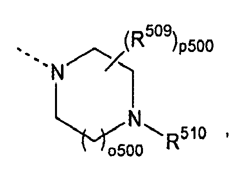 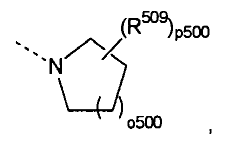 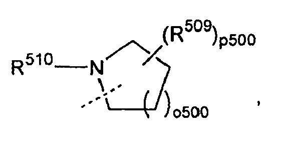 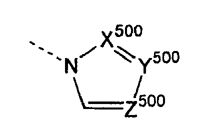 or 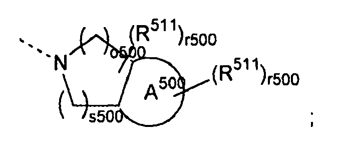
R⁵⁰⁷ and R⁵⁰⁸ are independently from each other selected from
H,
C₁₋₆-alkyl,
C₂₋₆-alkenyl,
C₂₋₆-alkinyl, and
C₂₋₆-alkylene-O-C₁₋₆-alkyl,
wherein each alkyl, alkenyl and alkinyl is optionally substituted by one or
more halogen atoms, CN or OH;
R⁵⁰⁹ is independently selected from
halogen,
CN,
OH,
C₁₋₆-alkyl optionally substituted with 1 to 3 substituents selected from halogen, CN and OH, and
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH;
R⁵¹⁰ is H, or
C₁-C₆-alkyl;
R⁵¹¹ is independently selected from
halogen,
CN,
OH,
C₁₋₆-alkyl optionally substituted with 1 to 3 substituents selected from halogen, CN and OH,
O-C₁₋₆-alkyl optionally substituted with 1 to 3 substituents selected from halogen, CN and OH,
C₁₋₆-alkylene-O-C₁₋₆-alkyl optionally substituted with 1 to 3 substituents selected from halogen, CN and OH,
-NH₂,
-NH(C₁₋₆-alkyl), and
-N(C₁₋₆-alkyl)₂;
X⁵⁰⁰ is CH or N;
Y⁵⁰⁰ is CH or N;
Z⁵⁰⁰ is CH or N;
A⁵⁰⁰ is a 3-7-membered saturated, unsaturated or aromatic ring containing 0-2 nitrogen atoms;
B⁵⁰⁰ is CR⁵⁰² or N;
G⁵⁰⁰ is CR⁵⁰² or N;
D⁵⁰⁰ is CR⁵⁰² or N;
E⁵⁰⁰ is CR⁵⁰² or N;
with the proviso that one or two of the variables B⁵⁰⁰, G⁵⁰⁰, D⁵⁰⁰ and E⁵⁰⁰ must be N;
R⁵⁰² is independently selected from
H,
F,
Cl,
CH₃,
OCH₃, and
CF₃;
R⁵⁰³ is H,
Cl,
F, or
CH₃;
R⁵⁰⁴ is Cl or F;
R⁵⁰⁵ is 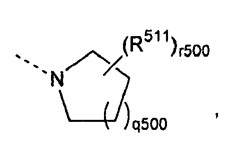 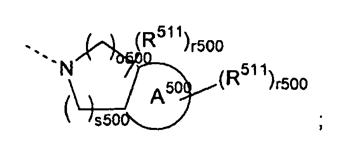 morpholine, optionally substituted by 1 to 3, same or different substituents R⁵¹⁴, or
NR⁵¹²R⁵¹³;
R⁵¹² and R⁵¹³ are independently from each other selected from
C₁₋₆-alkyl,
C₂₋₆-alkenyl,
C₂₋₆-alkinyl,
C₂₋₆-alkylene-O-C₁₋₆-alkyl, and
C₂₋₆-alkylene-N-(C₁₋₆-alkyl)₂;
R⁵¹⁴ is C₁₋₆-alkyl,
C₁₋₆-alkylene-O-C₁₋₆-alkyl,
C₁₋₆-alkylene-OH,
C₁₋₆-alkylene-NH₂,
C₁₋₆-alkylene-NH-C₁₋₆-alkyl, or C₁₋₆-alkylene-N(C₁₋₆-alkyl)₂;
l500 is 0, 1, 2, 3, or 4;
m500 is 0, 1, 2, 3, or 4;
o500 is 0, 1, or 2;
p500 is 0, 1, 2, 3, or 4;
q500 is 0, 1, 2, or 3;
r500 is 0, 1, 2, 3, or 4 and
s500 is 1, or 2.

14. The use according to any of claims 8 to 13 wherein the melanocortin-4 receptor antagonists is combined with one or more further therapeutic agent(s) selected from phenotiazines, 5HT3 receptor antagonists, dopamine antagonists, anticholinergic agents, anti histamins, histamine analogues, cannabinoids, corticosteroids, GABA receptor antagonists, NK1 receptor antagonists, alpha2 and alpha3 adrenoceptor antagonists.
